# EUROPEAN PATENT APPLICATION

(11) **EP 2 586 866 A1**
(43) Date of publication of application: **01.05.2013**
(21) Application number: 12166846.1
(22) Date of filing: 09.05.2008
(51) Int. Cl.: C12N 15/82, C12N 15/11, C12N 15/12, C12N 5/10, A01N 25/00, A01H 5/00

(54) **A method for sustainable transgene transcription**

(30) Priority: 09.05.2007 US 928287 P
(62) Divisional of application: 08759495.8
(71) Applicant: Devgen NV, 9030 Zwijnaarde (BE)
(72) Inventor: Oosthuyse, Bert, Wim, 8710 Ooigem (BE)
(74) Representative: De Clercq, Ann G. Y.

(57) **Abstract**

The present invention relates to constructs and methods for improving expression of transgenes in plants, animals and humans by introducing introns into the transcript to limit the exon sizes to less than about 60 bp.

SEQUENCE LISTING

<110>  Devgen NV

<120>  A method for sustainable transgene transcription

<130>  DEV-038-EP-DIV1

<150>  US 60/928,287
<151>  2007-05-09

<160>  17

<170>  PatentIn version 3.5

<210>  1
<211>  607
<212>  DNA
<213>  Leptinotarsa decemlineata

<400>  1
cgcagatcaa gcatatgatg gctttcattg aacaagaggc aaacgaaaag gcagaagaaa      60

tcgatgccaa ggccgaggaa gaatttaata ttgaaaaggg gcgccttgtt cagcaacaac     120

gtctcaagat tatggaatat tatgagaaga aagagaaaca ggtcgaactc cagaaaaaaa     180

tccaatcgtc taacatgttg aatcaggctc gattgaaagt attgaaggtt agggaagatc     240

acgttcgtac cgtactagag gaggcgcgta aacgacttgg tcaggtcaca aacgaccagg     300

gaaaatattc ccaaatcctg gaaagcctca ttttgcaggg attatatcag ctttttgaga     360

aagatgttac cattcgagtt cggccccagg accgagaact ggtcaaatcc atcattccca     420

ccgtcacgaa caagtataaa gatgccaccg gtaaggacat ccatctgaaa attgatgacg     480

aaatccatct gtcccaagaa accaccgggg gaatcgacct gctggcgcag aaaaacaaaa     540

tcaagatcag caatactatg gaggctcgtc tggagctgat ttcgcagcaa cttctgcccg     600

agatccg                                                               607


<210>  2
<211>  201
<212>  PRT
<213>  Leptinotarsa decemlineata

<400>  2

Gln Ile Lys His Met Met Ala Phe Ile Glu Gln Glu Ala Asn Glu Lys
1               5                   10                  15


Ala Glu Glu Ile Asp Ala Lys Ala Glu Glu Glu Phe Asn Ile Glu Lys
            20                  25                  30


Gly Arg Leu Val Gln Gln Gln Arg Leu Lys Ile Met Glu Tyr Tyr Glu
        35                  40                  45


Lys Lys Glu Lys Gln Val Glu Leu Gln Lys Lys Ile Gln Ser Ser Asn
    50                  55                  60

Met Leu Asn Gln Ala Arg Leu Lys Val Leu Lys Val Arg Glu Asp His
65                  70                  75                  80

Val Arg Thr Val Leu Glu Glu Ala Arg Lys Arg Leu Gly Gln Val Thr
                85                  90                  95

Asn Asp Gln Gly Lys Tyr Ser Gln Ile Leu Glu Ser Leu Ile Leu Gln
            100                 105                 110

Gly Leu Tyr Gln Leu Phe Glu Lys Asp Val Thr Ile Arg Val Arg Pro
        115                 120                 125

Gln Asp Arg Glu Leu Val Lys Ser Ile Ile Pro Thr Val Thr Asn Lys
    130                 135                 140

Tyr Lys Asp Ala Thr Gly Lys Asp Ile His Leu Lys Ile Asp Asp Glu
145                 150                 155                 160

Ile His Leu Ser Gln Glu Thr Thr Gly Gly Ile Asp Leu Leu Ala Gln
                165                 170                 175

Lys Asn Lys Ile Lys Ile Ser Asn Thr Met Glu Ala Arg Leu Glu Leu
            180                 185                 190

Ile Ser Gln Gln Leu Leu Pro Glu Ile
        195                 200

<210>  3
<211>  63
<212>  DNA
<213>  Leptinotarsa decemlineata

<400>  3
gggaagatca cgttcgtacc gtactagagg aggcgcgtaa acgacttggt caggtcacaa      60

acg                                                                    63

<210>  4
<211>  167
<212>  DNA
<213>  Leptinotarsa decemlineata

<400>  4
ttaattaagg gaagatcacg ttcgtaccgt actagaggag gcgcgtaaac gacttggtca      60

ggtcacaaac gacctaggat gaacgacatg ccatacgacg tttgtgacct gaccaagtcg     120

tttacgcgcc tcctctagta cggtacgaac gtgatcttcc cactagt                   167

<210>  5
<211>  80
<212>  DNA

<213>  Artificial sequence

<220>
<223>  SI

<400>  5
gtaagatata ttcttcgatt gccattattt ctctgttacg attatctgaa gattctctga      60

ttctatttct ttatttgcag                                                  80


<210>  6
<211>  80
<212>  DNA
<213>  Artificial sequence

<220>
<223>  SI

<400>  6
gtaagtttta ctgacttatg atttctctcg cattctcttc tgatttcgat ttcttatttc      60

ctattcttgt atttattcag                                                  80


<210>  7
<211>  80
<212>  DNA
<213>  Artificial sequence

<220>
<223>  SI

<400>  7
gtaagtatcc atttgcatat attcttccat cggatttcta atctgaattc tgcttactga      60

ctctaattat tttattgcag                                                  80


<210>  8
<211>  80
<212>  DNA
<213>  Artificial sequence

<220>
<223>  SI

<400>  8
gtaagatatg attctctatt tgcttgattc tttatcgatt cctatctgac tgatttctct      60

catgatttct ttatttgcag                                                  80


<210>  9
<211>  80
<212>  DNA
<213>  Artificial sequence

<220>
<223>  SI

<400>  9
gtaagtatat ctgatttctc tctttgagat tcttatcctg atctgattta ttccatggaa      60

tttctcttat tttattgcag                                                  80

<210>  10
<211>  80
<212>  DNA
<213>  Artificial sequence

<220>
<223>  SI

<400>  10
gtaagtttct tatatatgga tccatttata tacgttcttc tgatcgcttt tatctctgat      60

ctgaatttgt tttttttcag                                                  80


<210>  11
<211>  80
<212>  DNA
<213>  Artificial sequence

<220>
<223>  SI

<400>  11
gtaagtattt cagatctttc ttgccatata cgatttatcc atctgatttc tgaatcttca      60

ccattcttat tttattgcag                                                  80


<210>  12
<211>  80
<212>  DNA
<213>  Artificial sequence

<220>
<223>  SI

<400>  12
gtaagtttat gctttctaat gcttattctt cctatctttc tgattacact tgaattctta      60

cttcgattat tctttttcag                                                  80


<210>  13
<211>  80
<212>  DNA
<213>  Artificial sequence

<220>
<223>  SI

<400>  13
gtaagatact tcttacgata tattcgatct cttatctgat tccttctgat tattctctat      60

ttgctattct ttatttgcag                                                  80


<210>  14
<211>  887
<212>  DNA
<213>  Artificial sequence

<220>
<223>  target LD14 mexonized hairpin

<400>  14
ttaattaagg taagatatat tcttcgattg ccattatttc tctgttacga ttatctgaag      60

attctctgat tctatttctt tatttgcagg gaagatcacg ttcgtaagtt ttactgactt     120

atgatttctc tcgcattctc ttctgatttc gatttcttat ttcctattct tgtatttatt     180

caggtaccgt actagaggta agtatccatt tgcatatatt cttccatcgg atttctaatc     240

tgaattctgc ttactgactc taattatttt attgcaggag gcgcgtaaac ggtaagatat     300

gattctctat ttgcttgatt ctttatcgat tcctatctga ctgatttctc tcatgatttc     360

tttatttgca gacttggtca ggtaagtata tctgatttct ctctttgaga ttcttatcct     420

gatctgattt attccatgga atttctctta ttttattgca ggtcacaaac gacctaggat     480

gaacgacatg ccatacgacg tttgtgacct ggtaagtttc ttatatatgg atccatttat     540

atacgttctt ctgatcgctt ttatctctga tctgaatttg ttttttttca gaccaagtcg     600

tttacggtaa gtatttcaga tctttcttgc catatacgat ttatccatct gatttctgaa     660

tcttcaccat tcttatttta ttgcagcgcc tcctctagta cggtaagttt atgctttcta     720

atgcttattc ttcctatctt tctgattaca cttgaattct tacttcgatt attctttttc     780

aggtacgaac gtgatctgta agatacttct tacgatatat tcgatctctt atctgattcc     840

ttctgattat tctctatttg ctattcttta tttgcagtcc cactagt                   887

<210>  15
<211>  20
<212>  DNA
<213>  Artificial sequence

<220>
<223>  oGAU557

<400>  15
ctcggattcc attgcccagc                                                  20

<210>  16
<211>  21
<212>  DNA
<213>  Artificial sequence

<220>
<223>  oGBM276

<400>  16
cgtatggcat gtcgttcatc c                                                21

<210>  17
<211>  25
<212>  DNA
<213>  Artificial sequence

<220>
<223>  oGBM467

<400> 17
cgttaattaa gggaagatca cgttc 25

## Description

### Field of the invention

The present invention relates to the field of molecular biology, more specific to the expression of genes and transgenes in plants, animals and humans.

### Background to the invention

In this post-genomic era, genetic transformation of plants has become a widely used technology that serves multiple purposes in the fields of commerce and research. The use of transgene technology allows the improvement of certain plant traits including disease resistance, stress tolerance and enhanced nutrition. In addition, the use of transgenic plants for the production of various high-value proteins is becoming increasingly important. Transgenic plants are also frequently used in fundamental research as a tool to study gene function by overexpressing the target genes or knock-down of target gene expression.

One application of transgene technology in plants is expression of proteins encoded by a transgene. In the art of plant genetics, it has now become common practice to create genetically engineered plants, referred to as transgenic plants, which have stably inserted into their chromosomes one or more gene constructions intended to express an endogenous, a novel or foreign protein in the transgenic plants. Techniques exist to insert genes into plant cells and to regenerate whole fertile transgenic plants from such cells. For several important commercial crop species, transgenic seeds are commercially available and are widely planted and harvested.

Another application is RNA interference (RNAi) for endogenous gene knock down via expression of a double-stranded RNA (dsRNA) molecule. RNAi is a post-transcriptional process of sequence-specific down-regulation of gene expression (also referred to as "gene silencing" or "RNA-mediated gene silencing" or "post-transcriptional silencing (PTGS)) and is initiated by dsRNA that is complementary in sequence to a region of the target gene messenger RNA (mRNA) to be down-regulated. RNAi finds its applications both in agriculture, e.g. in plants, as in therapeutics.

Another application of transgene technology in plants is RNAi for plant pest and disease control. Here, the transgene expresses a dsRNA that is specific to a target RNA in the pest or disease organism. This so-called 'trans-species' RNAi results in gene knock down upon feeding by the pest or uptake by the disease organism on the transgenic plant tissues.

Transgene expression can be inhibited at the transcriptional level or the post-transcriptional level,

Transgenic events in plants often suffer from what is called transcriptional gene silencing (TGS) of the transgene. The process of TGS is associated with methylation of the DNA of the transgene and a dense chromatin structure which prevents the transcription of the transgene. DNA methylation of nuclear target sequences is mediated by corresponding small interfering RNA (siRNA) molecules and is dependent on homology of the siRNA sequence to the transgene. These siRNA molecules are the result of dsRNA cleavage by enzymes of the RNAi machinery and are about 21 to 26 or 27 nucleotides in length. A source of dsRNA molecules can be the transcription of aberrant RNA molecules from the integrated transgene. E.g. in the case of complex transgenic events that have multiple copies of the transgene integrated at a particular location in the plant genomes, the activity of the transgene promoter or from neighbouring endogenous plant promoters may drive the transcription of aberrant RNA molecules containing dsRNA fragments of the transgene construct. Other transgene constructs, e.g. constructs designed for gene silencing, are vulnerable to transcriptional gene silencing due to the nature of such constructs; most of these constructs drive the transcription of a double stranded hairpin RNA molecule.

dsRNA containing stretches of 28 contiguous nucleotides that are identical to the nuclear GFP transgene is sufficient to induce de novo methylation of the the GFP transgene (Thomas et al., The Plant Journal, 2001, 25(4), 417-425). DNA targets of 60 contiguous nucleotides with 100% sequence identity to dsRNA fragments are prone for de novo methylation. Stretches of 30 nucleotides have been shown to be suffient for RNA-directed DNA methylation, albeit less efficient (Pelissier and Wassenegger, RNA, 2000, 6, 55-65).

Transcriptional gene silencing (TGS) of transgenes is an undesirable side effect which frequently occurs in transgenic plants. In particular, transgenic plants that express dsRNA to silence endogenous genes by RNAi may loose their silencing capacity in next generations, as a result of absence of homologous siRNA/dsRNA production. For 'trans-species' RNAi, TGS of the transgene is absolutely to be avoided as the success of such an approach is absolutely dependent on a good and durable production of siRNA/dsRNA molecules.

The ability to manipulate the gene silencing pathways provides significant advantages in the field of biotechnology. Numerous methods are known in the art to stably and efficiently silence genes. However, nothing is known on methods and compositions to prevent gene-silencing. Especially in the field of plant transformation, novel methods and compositions for sustainable expression of the transgene over next generations are therefore needed. As for plants, likewise, therapeutic applications of RNAi in stem cell therapy would benefit from lifetime high expression levels of short hairpin RNA molecules by avoidance of TGS. Similarly, the success of transgene-mediated RNAi in livestock, e.g. as a prophylactic RNAi-mediated strategy to conquer, for instance, the bird flu H5N1, will depend on sustainable expression levels of RNAi molecules over generations.

### Description of the invention

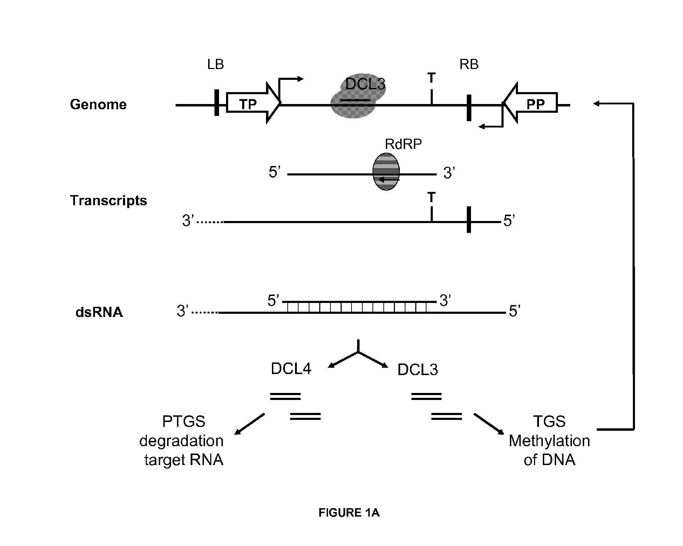

Until now, it has been difficult to avoid methylation of inserted transgene sequences, which causes modifications of histon proteins and condensation of the genomic DNA at the site of integration resulting in transcriptional gene silencing. Transgene methylation is caused by the expression of transcripts that result in stretches of double stranded RNA that are homologous to the parts of the integrated transgene construct. This can be the result of expression of aberrant transcript, or can be due to tail-to-tail insertion of copies of the transgene, or due to inherent complementarity in the sequences that are transcribed. These dsRNAs are recognized by dicer-like RNAseIII enzymes to be processed into siRNAs, typically 21 to 26 or 27 nucleotides in length. These siRNA molecules guide the RNA-inducing transcriptional silencing complex (RITS) to homologous nuclear sequences of the transgene construct to induce methylation of the DNA. An example of a transgene that is subject to transcriptional gene silencing is given in Figure 1A. The activity of RNA-dependent RNA polymerases (RdRP) represents an extra source of production of dsRNA of the transgene transcript. RNAi silencing constructs form a special class of transgenes that are susceptable to transcriptional gene silencing as these transgenes are especially designed to produce dsRNA.

However, the present inventors identified a means for avoiding the transcriptional gene silencing, such as in a plant generation, based on the introduction of introns in the transgene or in the inserted sequence to be transcribed. The introduction of introns to boost expression has been reported (Rose, (2002) Requirements for intron-mediated enhancement of gene expression in Arabidopsis, RNA, 8: 1444-1453). However, introns in the present invention serve other purposes then expression boosts.

The present invention relates to constructs, transgene expression cassettes and methods to make or adapt transgene expression cassettes by including introns in the transcript to limit the exon sizes to less than about 60 bp, preferably less than about 40 bp or 30 bp, more preferred less than the size of siRNA (21 to 26 or 27 bp) and even more preferred less than 20 bp, 19 bp, 18 bp, 17 bp, 16 bp, 15 bp, 14 bp, 13 bp, 12 bp, 11 bp or even less than 10 bp. The resulting spliced transgene transcript will not contain stretches of contiguous nucleotides with a sequence identical to the originally integrated nuclear transgene DNA sequence, said stretches being not longer than 59 nucleotides preferably not longer than 49 nucleotides, 39 nucleotides or 29 nucleotides, even more preferred not equal or longer than 21 to 26 or 27 nucleotides, even still more preferred less than 20 bp, 19 bp, 18 bp, 17 bp, 16 bp, 15 bp, 14 bp, 13 bp, 12 bp, 11 bp or even less than 10 bp. In the occurrence of dsRNA formation of the transgene transcript, this dsRNA does not contain stretches of contiguous nucleotides with a sequence indentical to the nuclear transgene DNA sequence, said stretches being not longer than 59 bp preferably not longer than 49 bp, 39 bp or 29 bp and even more preferred not equal or longer than the size of siRNA (e.g. 21 to 26 or 27 bp), ie not longer than 21 bp, said stretches preferably being about 10 bp long.

Even more preferred is that dsRNA-derived dicer products, siRNAs, do not have a sequence 100 % identical to the nuclear transgene DNA sequence, preferably the % of identity between the produced siRNAs and stretches of contiguous nucleotides in the nuclear transgene sequence is less than 100%, 99%, 98%, 97%, 96%, 95%, 94%, 93%, 92%, 91%, 90%, 89%, 88%, 87%, 86%, 85% or 80%, even more preferred less than 75%, 70%, 65% or 60% and most preferred less than 55% or 50%.

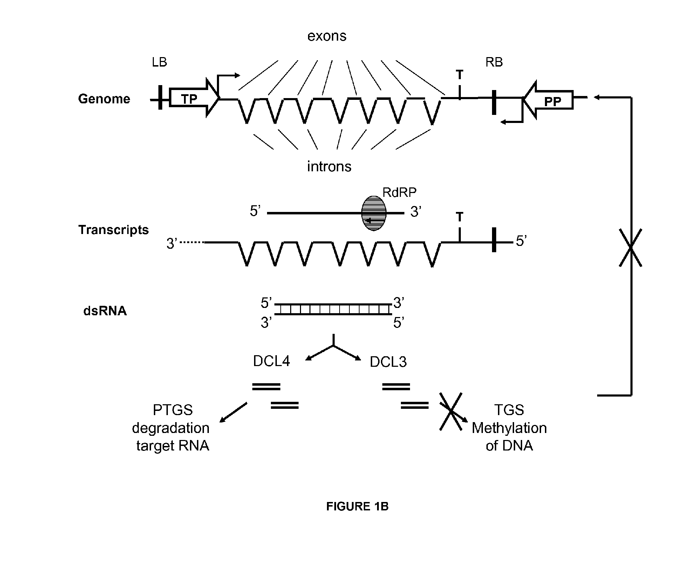

An example of a modified transgene construct is given in Figure 1B. The transgene is modified to contain mini-exons or 'mexons' of less than 60 bp long, most preferably the exon length 10-15 nucleotides long, through the insertion of introns. The general characteristics of introns such as splice donors, splice acceptors and splice branch sites are described by Lim and Burge (2001, PNAS. 98: 11193-11198). As a result of these modifications, the mRNA of the transgene is formed through splicing out of the intron sequences. In the occurrence of dsRNA formation, e.g. through the action of RdRP or basepairing with aberrant transgene transcripts, methylation of the nuclear transgene construct DNA sequences and transcriptional gene silencing is not induced because the dsRNA lacks the degree of sequence homology. The spliced products will share only less than 60 contiguous nucleotides of sequence homology, preferably less than 10-15 contiguous nucleotides of sequence homology, with the original nuclear transgene DNA.

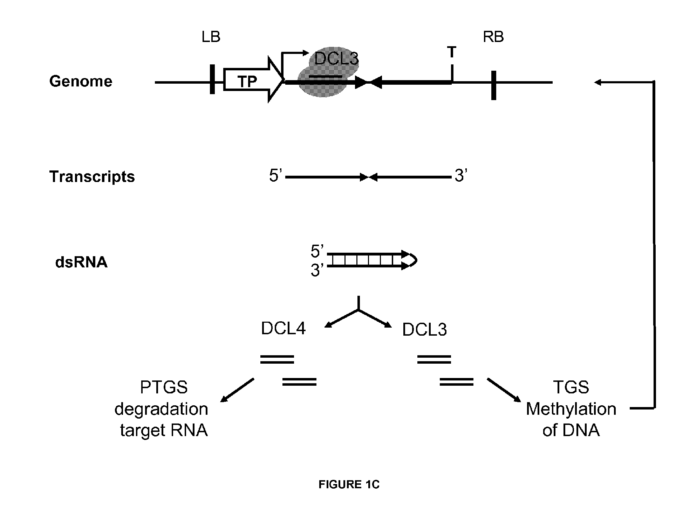

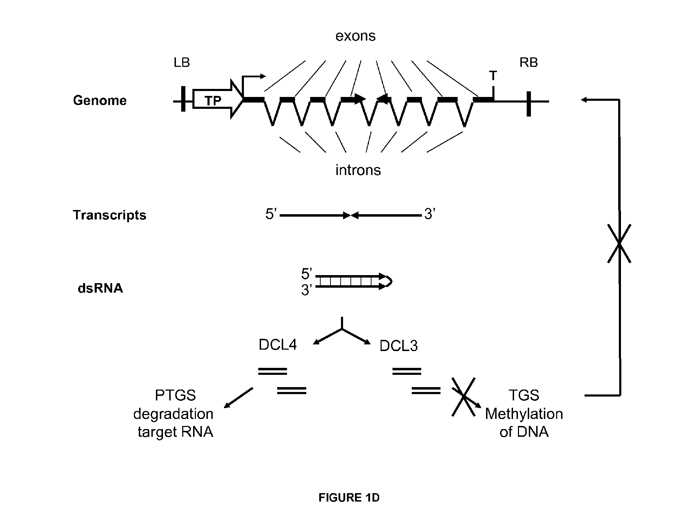

Expression constructs to transcribe hairpins are by nature of their construct extremely vulnerable to DNA methylation when integrated into the plant genome. Transcripts from hairpin constructs form dsRNA which is processed by dicer into the typical 21 to 26 or 27 nucleotides long siRNA molecules, capable to guide DNA methylation factors towards the nuclear homologues sequences and induce DNA methylation and transcriptional gene silencing (Figure 1C). In the case of trans-species RNAi where a pest organism needs to ingest RNAi effector molecules (dsRNA) produced and present whithin the plant, a transcriptional gene silencing of the RNAi expression construct is undesirable. However, limiting the size of the exons in the transcript eliminates the necessary homology at the siRNA level between the spliced hairpin sequence and the original nuclear transgene sequence (Figure 1 D).

In another embodiment of the herein described invention, similar as for plants, short hairpin RNA (shRNA) transgenes for livestock engineering may be 'mexonized' through the inclusion of one or more introns in sense and antisense target sequences. In such way, sequence homology between the resulting siRNA molecule and the nuclear shRNA transgene is reduced to a level such that siRNA-mediated epigenetic modification of the transgene is circumvented. For instance, a strategy of prophylactic application of RNAi to protect poultry against the avian influenza H5N1 will depend on durable expression levels of the siRNA effector molecules, over the bird's lifetime and over generations (Graeme O'Neill, 2007. Australia tackles bird flu using RNAi. Nature Biotechnology, Vol. 25, p605-606). 'Mexonization' of the selected H5N1 target sequences in the shRNA through the inclusion of one or more introns in both the sense and antisense target sequence of the shRNA will result in reduced contiguous sequence homology between the nuclear transgene sequence and the spliced expressed shRNA. siRNA, resulting from dicer activity on the shRNA, will lack the required homology to find the nuclear counterpart 'mexonized' transgene DNA. In such way, siRNA-mediated epigenetic modification of the transgene is omitted.

Still another embodiment of the invention finds its application in therapeutic applications, such as stem cell therapy. Here, genetic manipulation of human stem cells leads to prevention and treatment of human diseases (Grimm and Kay, 2007. RNAi and Gene Therapy: A Mutual attraction. Hematology, p473-481). Long-lasting, lifetime effect of genetic engineered stem cells in the patient will depend on durable expression of the introduced transgene. For instance, an RNAi strategy to protect lymphocytes from HIV infection, relies on good and durable expression levels of RNAi effector molecules in the blood cells, preferably for life. 'Mexonization' of the selected HIV target sequences in the shRNA through the inclusion of 1 or more introns in both the sense and antisense target sequence of the shRNA will result in reduced contiguous sequence homology between the nuclear transgene sequence and the spliced expressed shRNA. siRNA, resulting from dicer activity on the shRNA, will lack the required homology to find the nuclear counterpart 'mexonized' transgene DNA. In such way, the transgene in the genetic engineered hematopoietic stem cells will not be subject to siRNA-mediated epigenetic modification.

Any one of the applications and/or methods described herein results in that due to its structure, the (to be) transcribed transgene will not be recognized by the transcript-derived siRNAs and hence will not be methylated or silenced.

The methylation status of transgene constructs versus 'mexonized' constructs in transgenic cells, plants or animals or human can be analyzed by bisulphite sequencing of genomic DNA of the corresponding cells, plants or animals. For instance, this can be done by treatment of the genomic DNA of the plants with bisulphite to convert any unmethylated cytosine into uracil. Subsequent sequence analysis of the treated DNA will reveal the presence or absence of DNA methylation patterns in the transgene.

All technical terms employed in this specification and all molecular DNA cloning techniques and transformation protocols are commonly used in biochemistry, molecular biology and agriculture; hence, they are understood by those skilled in the field to which this invention belongs. Those technical terms and methods can be found, for example in: MOLECULAR CLONING: A LABORATORY MANUAL, 3rd ed., vol. 1-3, ed. Sambrook and Russel, Cold Spring Harbor Laboratory Press, Cold Spring Harbor, N.Y., 2001; CURRENT PROTOCOLS IN MOLECULAR BIOLOGY, ed. Ausubel et al., Greene Publishing Associates and Wiley-Interscience, New York, 1988 (with periodic updates); SHORT PROTOCOLS IN MOLECULAR BIOLOGY: A COMPENDIUM OF METHODS FROM CURRENT PROTOCOLS IN MOLECULAR BIOLOGY, 5th ed., vol. 1-2, ed. Ausubel et al., John Wiley & Sons, Inc., 2002; GENOME ANALYSIS: A LABORATORY MANUAL, vol. 1-2, ed. Green et al., Cold Spring Harbor Laboratory Press, Cold Spring Harbor, N.Y., 1997.

Methodology involving plant biology techniques are described here and also are described in detail in treatises such as METHODS IN PLANT MOLECULAR BIOLOGY: A LABORATORY COURSE MANUAL, ed. Maliga et al., Cold Spring Harbor Laboratory Press, Cold Spring Harbor, N.Y., 1995. Various techniques using PCR are described, for example, in Innis et al., PCR PROTOCOLS: A GUIDE TO METHODS AND APPLICATIONS, Academic Press, San Diego, 1990 and in Dieffenbach and Dveksler, PCR PRIMER: A LABORATORY MANUAL, 2nd ed., Cold Spring Harbor Laboratory Press, Cold Spring Harbor, N.Y., 2003. PCR-primer pairs can be derived from known sequences by known techniques such as using computer programs intended for that purpose, e.g., Primer, Version 0.5, 1991, Whitehead Institute for Biomedical Research, Cambridge, MA. Methods for chemical synthesis of nucleic acids are discussed, for example, in Beaucage & Caruthers, Tetra. Letts. 22: 1859-62 (1981), and Matteucci & Caruthers, J. Am. Chem. Soc. 103: 3185 (1981).

In the context of this disclosure, a number of terms and expressions will be utilized.

If not indicated otherwise, the term "expression" or "expressing" as used herein refers to the transcription of a polynucleotide. Alternativily, the term "expression" may also refer to the translation of mRNA into a polypeptide, which is more frequently used in "protein expression".

In a chromosomal environment, a gene contains coding regions that are interrupted by one or more non-coding regions (introns). An exon is any region of DNA within a gene that is transcribed to the final messenger RNA (mRNA) molecule, rather than being spliced out from the transcribed RNA molecule. Exons of many eukaryotic genes interleave with segments of non-coding DNA (introns). In many genes, each exon contains part of the open reading frame (ORF) that codes for a specific portion of the complete protein. However, the term *exon* is often misused to refer only to coding sequences for the final protein. This is incorrect, since many noncoding exons are known in human genes (Zhang 1998, Hum Mol Genet 7 (5): 919-32). Some of the exons will be wholly or part of the 5' untranslated region (5' UTR) or the 3' untranslated region (3' UTR) of each transcript. The untranslated regions are important for efficient translation of the transcript and for controlling the rate of translation and half life of the transcript. An intron is a portion of a gene that is transcribed into RNA, but subsequently removed from within the transcript prior to translation.

The expressions "gene suppression" or "down-regulation of gene expression" or "inhibition of gene expression" are used interchangeably and refer to a measurable or observable reduction in gene expression or a complete abolition of detectable gene expression, at the level of protein product and/or mRNA product from the target gene. Down-regulation or inhibition of gene expression is "specific" when down-regulation or inhibition of the target gene occurs without manifest effects on other genes of the pest. Depending on the nature of the target gene, down-regulation or inhibition of gene expression in cells of a pest can be confirmed by phenotypic analysis of the cell or the whole pest or by measurement of mRNA or protein expression using molecular techniques such as RNA solution hybridization, nuclease protection, Northern hybridization, reverse transcription quantitative PCR, gene expression monitoring with a microarray, antibody binding, enzymelinked immunosorbent assay (ELISA), Western blotting, radioimmunoassay (RIA), other immunoassays, or fluorescence-activated cell analysis (FACS).

The term "host cell" refers to a microorganism, a prokaryotic cell, a eukaryotic cell, a yeast cell, or a cell line cultured as a unicellular entity that may be, or has been, used as a recipient for a recombinant vector or other transfer of polynucleotides, and includes the progeny of the original cell that has been transfected. The progeny of a single cell may not necessarily be completely identical in morphology or in genomic or total DNA complement as the original parent due to natural, accidental, or deliberate mutation.

The term "isolated" polynucleotide is one that (1) has been substantially separated or purified from other polynucleotides of the organism in which the polynucleotide naturally occurs, i.e., other chromosomal and extrachromosomal DNA and RNA, by conventional nucleic acid purifications methods or (2) if the polynucleotide is in its natural or in another environment, the polynucleotide has been altered by deliberate human intervention to a composition and/or place at a locus in the cell other than the locus native to thepolynucleotide. The term also embraces recombinant polynucleotides and chemically synthesized polynucleotides.

A "dicotyledonous plant (dicot)" is a flowering plant whose embryos have two seed halves or cotyledons, branching leaf veins, and flower parts in multiples of four or five. Examples of dicots include but are not limited to, *Eucalyptus, Populus, Liquidamber, Acacia,* teak, mahogany, cotton, tobacco, *Arabidopsis,* tomato, potato, sugar beet, broccoli, cassava, sweet potato, pepper, poinsettia, bean, alfalfa, soybean, carrot, strawberry, lettuce, oak, maple, walnut, rose, mint, squash, daisy, geranium, avocado, and cactus.

A "monocotyledonous plant (monocot)" is a flowering plant having embryos with one cotyledon or seed leaf, parallel leaf veins, and flower parts in multiples of three. Examples of monocots include, but are not limited to turfgrass, maize, rice, oat, wheat, barley, sorghum, millet, orchid, iris, lily, onion, and palm.

The terms "polynucleotide" and "nucleic acid" are used interchangeably herein. These terms encompass nucleotide sequences and the like. Unless otherwise defined herein, a polynucleotide may be a polymer of RNA or DNA that is single-or double stranded and can contain natural, synthetic, non-natural and/or altered nucleotide bases.

The term "pest" or "target pest" includes but is not limited to insects, arachnids, crustaceans, fungi, bacteria, viruses, nematodes, flatworms, roundworms, pinworms, hookworms, tapeworms, trypanosomes, schistosomes, botflies, fleas, ticks, mites, and lice that are pervasive in the human environment and infect, infest or damage plants or animals or other substances.

The term "pesticide" refers to any substance or mixture of substances intended for preventing, destroying, repelling, or mitigating any pest. A pesticide may be a chemical substance or biological agent, such as a transgenic plant, used against pests (see definition above) that compete with humans for food, destroy property, spread disease, or are a nuisance.

The term "progeny" as used in reference to the progeny of a transgenic plant, is one that is born of, begotten by, or derived from a plant or the transgenic plant. Thus, a "progeny" plant, i.e., an "F1" generation plant is an offspring or a descendant of the transgenic plant produced by the inventive methods. A progeny of a transgenic plant may contain in at least one, some, or all of its cell genomes, the desired polynucleotide that was integrated into a cell of the parent transgenic plant by the methods described herein. Thus, the desired polynucleotide is "transmitted" or "inherited" by the progeny plant. The term "progeny" as used herein, also may be considered to be the offspring or descendants of a group of plants.

The expression "commodity product" encompasses any product made or otherwise derived from a plant, including but not limited to food, feed, fiber, paper, meal, protein, starch, flour, silage, coffee, tea, and oil.

An "RNA transcript" refers to the product resulting from RNA polymerase-catalyzed transcription of a DNA sequence. "Messenger RNA (mRNA)" refers to the RNA that is without introns and that may be translated into protein by the cell. An RNA transcript is not necessarily translated into protein but may also reside in the cell for instance as a partially or complete double stranded RNA molecule. "Complementary DNA" (cDNA) refers to single-stranded DNA synthesized from a mature mRNA template. Though there are several methods, cDNA is most often synthesized from mature (fully spliced) mRNA using the enzyme reverse transcriptase. This enzyme operates on a single strand of mRNA, generating its complementary DNA based on the pairing of RNA base pairs (A, U, G, C) to their DNA complements (T, A, C, G). Two nucleic acid strands are "substantially complementary" when at least 85% of their bases pair.

The term "silencing" refers collectively to a variety of techniques, naturally occurring or not, used to suppress or turn off expression of a gene, so that the transcript or the product of the gene is not present or present at a reduced level in an organism that is below the level found in a control organism. As used herein, reduced level means decreased, reduced, lowered, prevented, inhibited, stopped, suppressed, eliminated, and the like. Reduced level includes expression that is decreased by about 10%, 15%, 20%, 25%, 30%, 35%, 40%, 45%, 50%, 55%, 60%, 65%, 70%, 75%, 80%, 85%, 90%, 95%, or 100% relative to the appropriate control organism. A reduction in the expression of a polynucleotide of interest may occur during and/or subsequent to growth of the organism (i.e., plant) to the desired stage of development. As described earlier, "TGS" and "PTGS" refers to a series of naturally occurring phenomena that reduce expression, i.e. transcription and/or translation, of genes.

"Splicing" is a modification of genetic information after transcription, in which introns are removed and exons are joined. Since in prokaryotic genomes introns do not exist, splicing naturally only occurs in eukaryotes. There splicing prepares the precursor messenger RNA (pre-mRNA) to produce the mature messenger RNA (mRNA), which then undergoes translation as part of the protein synthesis to produce proteins. Splicing includes a series of biochemical reactions, which are catalyzed by the spliceosome, a complex of small nuclear ribonucleo-proteins (snRNPs). The major spliceosome splices introns containing GU at the 5' splice site and AG at the 3' splice site.

The term "transcription" refers to the process of copying DNA to RNA by an enzyme called RNA polymerase.

The term "translation" is the second process of protein biosynthesis (part of the overall process of gene expression).Translation occurs in the cytoplasm where the ribosomes are located. In translation, messenger RNA (mRNA) is decoded to produce a specific polypeptide according to the rules specified by the genetic code. Translation is necessarily preceded by transcription.

A "transgene" is a gene or genetic material which has been transferred by any of a number of genetic engineering techniques from one organism to another. The other organism may be of the same or of a different species. In its most precise usage, the term transgene describes a segment of DNA containing a gene sequence which has been isolated from one organism and is introduced into a different organism. This non-native segment of DNA may retain the ability to produce RNA or protein in the transgenic organism or it may alter the normal function of the transgenic organism's genetic code. Typically the DNA is incorporated into the organism's germ line.

The term "mexon" as used herein relates to exons, e.g. mini-exons, created by the introduction of additional introns in gene sequences and resulting in exons of less than about 60 bp.

The term "mexonization of transgene constructs" as used herein refers to the method described herein to introduce introns in a transgene so that mini-exons are formed.

It is understood that the present invention is not limited to the particular methodology, protocols, vectors, and reagents, etc., described herein, as these may vary. It is also to be understood that the terminology used herein is used for the purpose of describing particular embodiments only, and is not intended to limit the scope of the present invention. It must be noted that as used herein and in the appended aspects, the singular forms "a," "an," and "the" include plural reference unless the context clearly dictates otherwise. Thus, for example, a reference to "a polynucleotide" is a reference to one or more polynucleotides, or "at least one" polynucleotide and includes equivalents thereof known to those skilled in the art and so forth.

In one embodiment of the invention, there is provided an isolated polynucleotide comprising a sequence to be transcribed, said sequence consisting of introns and exons wherein the size of the exons is smaller than 60 nucleotides. In particular embodiments, the number of exons present in the polynucleotide of the invention exceeds the naturally occurring number of exons in the genomic counterpart of the polynucleotide.

In a further embodiment, the isolated polynucleotide is such that the number of nucleotides in each of the exons independently ranges from 1 to 60 nucleotides, preferably from 1 to 55, from 1 to 50, from 1 to 40, from 1 to 30, from 1 to 26, from 1 to 21, from 1 to 15 or from 1 to 10, more preferably from 2 to 30, from 2 to 26, from 2 to 21, from 2 to 15 or from 2 to 10, from 3 to 30, from 3 to 26, from 3 to 21, from 3 to 15, from 3 to 10, from 4 to 30, from 4 to 26, from 4 to 21, from 4 to 15, from 4 to 10, from 5 to 30, from 5 to 26, from 5 to 21, from 5 to 15, from 5 to 10, most preferably from 10 to 30, from 10 to 26, from 10 to 21 or from 10 to 15 nucleotides.

In other embodiments the number of nucleotides in each of the exons independently ranges from 1 to 60, from 1 to 55, from 1 to 45, from 1 to 35, 1 to 29, from 1 to 28, from 1 to 27, from 1 to 25, from 1 to 24, from 1 to 23, from 1 to 22, from 1 to 20, from 1 to 19, from 1 to 18, from 1 to 17, from 1 to 16, from 1 to 14, from 1 to 13, from 1 to 12, from 1 to 11, from 1 to 9, from 1 to 8, from 1 to 7, from 1 to 6, from 1 to 5, from 1 to 4, from 1 to 3, from 1 to 2, from 2 to 60, from 2 to 55, from 2 to 50, from 2 to 45, from 2 to 40, from 2 to 35, from 2 to 29, from 2 to 28, from 2 to 27, from 2 to 25, from 2 to 24, from 2 to 23, from 2 to 22, from 2 to 20, from 2 to 19, from 2 to 18, from 2 to 17, from 2 to 16, from 2 to 14, from 2 to 13, from 2 to 12, from 2 to 11, from 2 to 9, from 2 to 8, from 2 to 7, from 2 to 6, from 2 to 5, from 2 to 4, from 2 to 3, from 3 to 60, from 3 to 55, from 3 to 50, from 3 to 45, from 3 to 40, from 3 to 35, from 3 to 29, from 3 to 28, from 3 to 27, from 3 to 25, from 3 to 24, from 3 to 23, from 3 to 22, from 3 to 20, from 3 to 19, from 3 to 18, from 3 to 17, from 3 to 16, from 3 to 14, from 3 to 13, from 3 to 12, from 3 to 11, from 3 to 9, from 3 to 8, from 3 to 7, from 3 to 6, from 3 to 5, from 3 to 4, from 4 to 60, from 4 to 55, from 4 to 50, from 4 to 45, from 4 to 40, from 4 to 35,from 4 to 29, from 4 to 28, from 4 to 27, from 4 to 25, from 4 to 24, from 4 to 23, from 4 to 22, from 4 to 20, from 4 to 19, from 4 to 18, from 4 to 17, from 4 to 16, from 4 to 14, from 4 to 13, from 4 to 12, from 4 to 11, from 4 to 9, from 4 to 8, from 4 to 7, from 4 to 6, from 4 to 5, from 5 to 60, from 5 to 55, from 5 to 50, from 5 to 45, from 5 to 40, from 5 to 35, from 5 to 29, from 5 to 28, from 5 to 27, from 5 to 25, from 5 to 24, from 5 to 23, from 5 to 22, from 5 to 20, from 5 to 19, from 5 to 18, from 5 to 17, from 5 to 16, from 5 to 14, from 5 to 13, from 5 to 12, from 5 to 11, from 5 to 9, from 5 to 8, from 5 to 7, from 5 to 6, from 6 to 60, from 6 to 55, from 6 to 50, from 6 to 45, from 6 to 40, from 6 to 35, from 6 to 30, from 6 to 29, from 6 to 28, from 6 to 27, from 6 to 25, from 6 to 24, from 6 to 23, from 6 to 22, from 6 to 21, from 6 to 20, from 6 to 19, from 6 to 18, from 6 to 17, from 6 to 16, from 6 to 15, from 6 to 14, from 6 to 13, from 6 to 12, from 6 to 11, from 6 to 10, from 6 to 9, from 6 to 8, from 6 to 7, from 7 to 60, from 7 to 55, from 7 to 50, from 7 to 45, from 7 to 40, from 7 to 35, from 7 to 30, from 7 to 29, from 7 to 28, from 7 to 27, from 7 to 25, from 7 to 24, from 7 to 23, from 7 to 22, from 7 to 20, from 7 to 21, from 7 to 19, from 7 to 18, from 7 to 17, from 7 to 16, from 7 to 15, from 7 to 14, from 7 to 13, from 7 to 12, from 7 to 11, from 7 to 10, from 7 to 9, from 7 to 8, from 8 to 60, from 8 to 55, from 8 to 50, from 8 to 45, from 8 to 40, from 8 to 35, from 8 to 30, from 8 to 29, from 8 to 28, from 8 to 27, from 8 to 25, from 8 to 24, from 8 to 23, from 8 to 22, from 8 to 21, from 8 to 20, from 8 to 19, from 8 to 18, from 8 to 17, from 8 to 16, from 8 to 15, from 8 to 14, from 8 to 13, from 8 to 12, from 8 to 11, from 8 to 10, from 8 to 9, from 9 to 60, from 9 to 55, from 9 to 50, from 9 to 45, from 9 to 40, from 9 to 35, from 9 to 30, from 9 to 29, from 9 to 28, from 9 to 27, from 9 to 26, from 9 to 25, from 9 to 24, from 9 to 23, from 9 to 22, from 9 to 21, from 9 to 20, from 9 to 19, from 9 to 18, from 9 to 17, from 9 to 16, from 9 to 15, from 9 to 14, from 9 to 13, from 9 to 12, from 9 to 11, from 9 to 10, from 10 to 60, from 10 to 55, from 10 to 50, from 10 to 45, from 10 to 40, from 10 to 35, from 10 to 30, from 10 to 29, from 10 to 28, from 10 to 27, from 10 to 26, from 10 to 25, from 10 to 24, from 10 to 23, from 10 to 22, from 10 to 21, from 10 to 20, from 10 to 19, from 10 to 18, from 10 to 17, from 10 to 16, from 10 to 15, from 10 to 14, from 10 to 13, from 10 to 12, from 10 to 11, from 11 to 60, from 11 to 55, from 11 to 50, from 11 to 45, from 11 to 40, from 11 to 35, from 11 to 30, from 11 to 29, from 11 to 28, from 11 to 27, from 11 to 26, from 11 to 25, from 11 to 24, from 11 to 23, from 11 to 22, from 11 to 21, from 11 to 20, from 11 to 19, from 11 to 18, from 11 to 17, from 11 to 16, from 11 to 15, from 11 to 14, from 11 to 13, from 11 to 12, from 12 to 60, from 12 to 55, from 12 to 50, from 12 to 45, from 12 to 40, from 12 to 35, from 12 to 30, from 12 to 29, from 12 to 28, from 12 to 27, from 12 to 26, from 12 to 25, from 12 to 24, from 12 to 23, from 12 to 22, from 12 to 21, from 12 to 20, from 12 to 19, from 12 to 18, from 12 to 17, from 12 to 16, from 12 to 15, from 12 to 14, from 12 to 13, from 13 to 60, from 13 to 55, from 13 to 50, from 13 to 45, from 13 to 40, from 13 to 35, from 13 to 30, from 13 to 29, from 13 to 28, from 13 to 27, from 13 to 26, from 13 to 25, from 13 to 24, from 13 to 23, from 13 to 22, from 13 to 21, from 13 to 20, from 13 to 19, from 13 to 18, from 13 to 17, from 13 to 16, from 13 to 15, from 13 to 14, from 14 to 60, from 14 to 55, from 14 to 50, from 14 to 45, from 14 to 40, from 14 to 35,from 14 to 30, from 14 to 29, from 14 to 28, from 14 to 27, from 14 to 26, from 14 to 25, from 14 to 24, from 14 to 23, from 14 to 22, from 14 to 21, from 14 to 20, from 14 to 19, from 14 to 18, from 14 to 17, from 14 to 16, from 14 to 15, from 15 to 60, from 15 to 55, from 15 to 50, from 15 to 45, from 15 to 40, from 15 to 35, from 15 to 30, from 15 to 29, from 15 to 28, from 15 to 27, from 15 to 26, from 15 to 25, from 15 to 24, from 15 to 23, from 15 to 22, from 15 to 21, from 15 to 20, from 15 to 19, from 15 to 18, from 15 to 17, from 15 to 16, from 16 to 60, from 16 to 55, from 16 to 50, from 16 to 45, from 16 to 40, from 16 to 35, from 16 to 30, from 16 to 29, from 16 to 28, from 16 to 27, from 16 to 26, from 16 to 25, from 16 to 24, from 16 to 23, from 16 to 22, from 16 to 21, from 16 to 20, from 16 to 19, from 16 to 18, from 16 to 17, from 17 to 60, from 17 to 55, from 17 to 50, from 17 to 45, from 17 to 40, from 17 to 35, from 17 to 30, from 17 to 29, from 17 to 28, from 17 to 27, from 17 to 26, from 17 to 25, from 17 to 24, from 17 to 23, from 17 to 22, from 17 to 21, from 17 to 20, from 17 to 19, from 17 to 18, from 18 to 60, from 18 to 55, from 18 to 50, from 18 to 45, from 18 to 40, from 18 to 35, from 18 to 30, from 18 to 29, from 18 to 28, from 18 to 27, from 18 to 26, from 18 to 25, from 18 to 24, from 18 to 23, from 18 to 22, from 18 to 21, from 18 to 20, from 18 to 19, from 19 to 60, from 19 to 55, from 19 to 50, from 19 to 45, from 19 to 40, from 19 to 35, from 19 to 30, from 19 to 29, from 19 to 28, from 19 to 27, from 19 to 26, from 19 to 25, from 19 to 24, from 19 to 23, from 19 to 22, from 19 to 21, from 19 to 20, from 20 to 60, from 20 to 55, from 20 to 50, from 20 to 45, from 20 to 40, from 20 to 35, from 20 to 30, from 20 to 29, from 20 to 28, from 20 to 27, from 20 to 26, from 20 to 25, from 20 to 24, from 20 to 23, from 20 to 22, from 20 to 21, from 21 to 60, from 21 to 55, from 21 to 50, from 21 to 45, from 21 to 40, from 21 to 35, from 21 to 30, from 21 to 29, from 21 to 28, from 21 to 27, from 21 to 26, from 21 to 25, from 21 to 24, from 21 to 23, from 21 to 22, from 22 to 60, from 22 to 55, from 22 to 50, from 22 to 45, from 22 to 40, from 22 to 35, from 22 to 30, from 22 to 29, from 22 to 28, from 22 to 27, from 22 to 26, from 22 to 25, from 22 to 24, from 22 to 23, from 22 to 22, from 22 to 21, from 23 to 60, from 23 to 55, from 23 to 50, from 23 to 45, from 23 to 40, from 23 to 35, from 23 to 30, from 23 to 29, from 23 to 28, from 23 to 27, from 23 to 26, from 23 to 25, from 23 to 24, from 24 to 60, from 24 to 55, from 24 to 50, from 24 to 45, from 24 to 40, from 24 to 35, from 24 to 30, from 24 to 29, from 24 to 28, from 24 to 27, from 24 to 26, from 24 to 25, from 25 to 60, from 25 to 55, from 25 to 50, from 25 to 45, from 25 to 40, from 25 to 35, from 25 to 30, from 25 to 29, from 25 to 28, from 25 to 27, from 25 to 26, from 26 to 60, from 26 to 55, from 26 to 50, from 26 to 45, from 26 to 40, from 26 to 35, from 26 to 30, from 26 to 29, from 26 to 28, from 26 to 27, from 27 to 60, from 27 to 55, from 27 to 50, from 27 to 45, from 27 to 40, from 27 to 35, from 27 to 30, from 27 to 29, from 27 to 28, from 28 to 60, from 28 to 55, from 28 to 50, from 28 to 45, from 28 to 40, from 28 to 35, from 28 to 30, from 28 to 29 or from 29 to 60, from 29 to 55, from 29 to 50, from 29 to 45, from 29 to 40, from 29 to 35, from 29 to 30 nucleotides.

In still another embodiment, the isolated polynucleotide is such that the number of nucleotides in each of the exons is independently chosen from the group comprising 60, 59, 58, 57, 56, 55, 54, 53, 52, 51, 50, 49, 48, 47, 46, 45, 44, 43, 42, 41, 40, 39, 38, 37, 36, 35, 34, 33, 32, 31, 30, 29, 28, 27, 26, 25, 24 or 23 nucleotides, preferably 22, 21, 20, 19, 18, 17, 16 or 15 nucleotides, more preferably 14, 13, 12, 11, 10, 9, 8, 7, 6, 5, 4, 3, 2 or 1 nucleotide(s).

As a source for introns, naturally occuring introns can be used, which can be retrieved from plant sequence databases or, alternatively, synthetic introns can be designed by someone skilled in the art, based on the consensus sequences of splice donor and acceptor sites, and the branch sites, as described in Lim and Burge (2001, PNAS. 98: 11193-11198).

In a further embodiment the present invention encompasses DNA constructs comprising a polynucleotide as described above operably linked to a transcriptional regulatory region regulating the transcription of the polynucleotide. The DNA construct may further comprise a transcriptional initiation site and/or a transcriptional termination site.

The transcriptional regulatory region may comprise for example a promoter that is located upstream of the polynucleotide (towards the 5' region, providing a control point for regulated gene transcription. Preferably said promoter is an RNA polymerase promoter directing transcription of the polynucleotide. In addition, the transcriptional regulatory region may comprise other regulatory regions (enhancers, silencers, boundary elements/insulators) that work in concert with promoters to direct the level of transcription of a given polynucleotide (or gene or transgene).

In one embodiment the exons of the DNA construct encode, upon transcription and splicing, a product of interest. A "product of interest" both relates to polypeptides or peptides transcribed and translated from the polynucleotide, and to RNA transcripts that are not translated.

The exons thus can include a sequence that expresses a gene of interest (e.g., an RNA encoding a protein), or a sequence that suppresses a gene of interest (e.g., an RNA that is processed to an siRNA or miRNA or dsRNA that in turn suppresses the gene of interest, said gene of interest can be in a trans organism).

A "gene of interest" can include any coding or non-coding sequence from any species (including, but not limited to, non-eukaryotes such as bacteria, and viruses; fungi; plants, including monocots and dicots, such as crop plants, ornamental plants, and non-domesticated or wild plants; invertebrates such as arthropods, annelids, nematodes, and molluscs; and vertebrates such as amphibians, fish, birds, and mammals). Non-limiting examples of a non-coding sequence to be expressed by a gene expression element include, but not limited to, 5' untranslated regions, promoters, enhancers, or other non-coding transcriptional regions, 3' untranslated regions, terminators, intron, microRNAs, microRNA precursor DNA sequences, small interfering RNAs, RNA components of ribosomes or ribozymes, small nucleolar RNAs, and other non-coding RNAs. Non-limiting examples of a gene of interest further include, but are not limited to, translatable (coding) sequence, such as genes encoding transcription factors and genes encoding enzymes involved in the biosynthesis or catabolism of molecules of interest (such as amino acids, fatty acids and other lipids, sugars and other carbohydrates, biological polymers, and secondary metabolites including alkaloids, terpenoids, polyketides, non-ribosomal peptides, and secondary metabolites of mixed biosynthetic origin). A gene of interest can be a gene native to the plant in which the DNA construct of the invention is to be transcribed, or can be a non-native gene. A gene of interest can be a marker gene, for example, a selectable marker gene encoding antibiotic, antifungal, or herbicide resistance, or a marker gene encoding an easily detectable trait (e.g., phytoene synthase or other genes imparting a particular pigment to the plant), or a gene encoding a detectable molecule, such as a fluorescent protein, luciferase, or a unique polypeptide or nucleic acid "tag" detectable by protein or nucleic acid detection methods, respectively). Selectable markers are genes of interest of particular utility in identifying successful processing of constructs of the invention.

In one embodiment, the genes of interest according to the invention are transgenes that encode proteins or polypeptides to be expressed.

In another embodiment, the genes of interest according to the invention are sequences that are at least in part complementary to a target gene in an organism, and wherein said target gene in said organism needs to be silenced or downregulated.

In a further embodiment the exons in the DNA construct of the present invention result upon transcription and splicing in a double stranded RNA consisting of:
(a) a first strand comprising a sequence substantially identical to 19 to 49, 19 to 39, 19 to 29, 19 to 25, 19 to 21; or 10 to 1000, 10 to 500, 10 to 50, 19 to 999, 19 to 499 or 19 to more than 1000 contiguous nucleotides of a target gene;
   or comprising a sequence that has at least 70%, preferably at least 75%, 80%, 85%, 90%, 95% or 99% sequence identity with a sequence comprising a 19 to 49, 19 to 39, 19 to 29, 19 to 25, 19 to 21; or 10 to 1000, 10 to 500, 10 to 50, 19 to 999, 19 to 499 or 19 to more than 1000 contiguous nucleotides of a target gene, and
(b) a second strand comprising a sequence substantially complementary to the first strand,
   wherein the double stranded RNA optionally having a single stranded overhang at either or both ends, and wherein the double stranded RNA inhibits expression of said target gene.

The target gene may be a gene in an organism such as an organism that infests, infects or damages plants or animals or feeds on for instance a plant or animal containing the polynucleotides or DNA constructs of the invention. Such an organism, e.g. a pest, may ingest or contact one or more cells or tissues, animals or plants, or products produced by a plant transformed with a polynucleotide or DNA construct of the invention. As a result of transcription and splicing, a double stranded RNA molecule is formed that inhibits the target gene in the pest organism, leading to lethality and/or mortality of the pest organism, thereby preventing that the pest organism further feeds on the cells, tissues, animals or plants; and/or preventing that the pest organism further infects, infests or damages the cells, tissues, animals or plants; and/or preventing that the infection or infestation further "spreads".

Pest organisms are chosen from the group comprising insects, arachnids, nematodes, viruses or fungi.

Preferred target genes are for instance the ones provided in WO2006/129204, WO2007/104570, WO2007/074405, WO2007/083193, WO 2007/080126 and WO2007/080127 by applicant, and WO2007/035650, or an orthologous gene identified in another pest organism.

The expression "sequence identity": as used herein, "sequence identity" or "identity" in the context of two nucleic acid sequences includes reference to the residues in the two sequences which are the same when aligned for maximum correspondence over a specified region. As used herein, percentage of sequence identity means the value determined by comparing two optimally aligned sequences over a comparison window, wherein the portion of the polynucleotide sequence in the comparison window may comprise additions or deletions (i.e., gaps) as compared to the reference sequence (which does not comprise additions or deletions) for optimal alignment of the two sequences. The percentage is calculated by determining the number of positions at which the identical nucleic acid base occurs in both sequences to yield the number of matched positions, dividing the number of matched positions by the total number of positions in the window of comparison and multiplying the result by 100 to yield the percentage of sequence identity. "Sequence identity" has an art-recognized meaning and can be calculated using published techniques. See COMPUTATIONAL MOLECULAR BIOLOGY, Lesk, ed. (Oxford University Press, 1988), BIOCOMPUTING: INFORMATICS AND GENOME PROJECTS, Smith, ed. (Academic Press, 1993), COMPUTER ANALYSIS OF SEQUENCE DATA, PART I, Griffin & Griffin, eds., (Humana Press, 1994), SEQUENCE ANALYSIS IN MOLECULAR BIOLOGY, Von Heinje ed., Academic Press (1987), SEQUENCE ANALYSIS PRIMER, Gribskov & Devereux, eds. (Macmillan Stockton Press, 1991), and Carillo & Lipton, SIAM J. Applied Math. 48: 1073 (1988). Methods commonly employed to determine identity or similarity between two sequences include but are not limited to those disclosed in GUIDE TO HUGE COMPUTERS, Bishop, ed., (Academic Press, 1994) and Carillo & Lipton, *supra.* Methods to determine identity and similarity are codified in computer programs such as the GCG program package (Devereux et al., Nucleic Acids Research 12: 387 (1984)), BLASTN, FASTA (Atschul et al., J. Mol. Biol. 215: 403 (1990)), and FASTDB (Brutlag et al., Comp. App. Biosci. 6: 237 (1990)). One preferred algorithm for determination of sequence identity is described in Dufresne et al., Nature Biotechnology, 2002, Vol. 20, 1269-1271.

Also encompassed herein is any RNA transcript encoded by any of the DNA constructs herein described.

The polynucleotides of the invention may be comprised in a vector that is used to transform a cell. Alternatively, the cell may be transformed with the polynucleotide or the DNA construct herein described. A recombinant nucleic acid vector may, for example, be a linear or a closed circular plasmid. The vector system may be a single vector or plasmid or two or more vectors or plasmids that together contain the total nucleic acid to be introduced into the genome of a prokaryotic or eukaryotic host. In addition, a the prokaryotic or eukaryotic vector may be an expression vector. Polynucleotides herein described can, for example, be suitably inserted into a vector under the control of a suitable promoter that functions in one or more I hosts, ie microbial, prokaryotic or eukaryotic, to drive expression of a linked coding sequence or other DNA sequence. Many vectors are available for this purpose. Each vector contains various components depending on its function (amplification of DNA or expression of DNA) and the particular host cell with which it is compatible. The expression "operably linked" in reference to a regulatory sequence and a structural nucleotide sequence, means that the regulatory sequence causes regulated expression of the linked structural nucleotide sequence. An expression vector for producing a mRNA can also contain an inducible promoter that is recognized by the host prokaryotic or eukaryotic organism and is operably linked to the nucleic acid encoding a gene or fragment of interest.

Other embodiments of the invention encompass prokaryotic or eukaryotic cells comprising any of the polynucleotides or the DNA constructs of the invention. Also encompassed is an eukaryotic cell comprising the polynucleotide or the DNA construct described herein integrated in its chromosome.

According to a more general aspect, the invention relates to a method for preventing TGS (transcriptional gene silencing) of a transgene in a plant comprising: (a) providing a polynucleotide consisting of a coding sequence to be transcribed optionally under the control of a transcriptional regulatory region, (b) introducing into the coding sequence splice intron sequences so that stretches of double stranded RNA formed from expression of the transcript are of a size not capable of base-pairing with the nuclear transgene expression cassette sequence necessary to conduct the DNA methylation process, (c) transforming a plant cell with the polynucleotide obtained in step (b) wherein said polynucleotide is operably linked to said transcriptional regulatory region or wherein said polynucleotide is inserted downstream and in frame of an (endogenous) transcriptional regulatory region regulating the transcription of the polynucleotide, and (d) regenerating a transgenic plant from said plant cell. The method may further comprise the step of evaluating whether correct transcription and optionally translation of the transgene has occurred.

Mexonized constructs may be applied for:
- Sustainable transgene expression for proteins
- Sustainable transcription of silencing constructs, i.e. constructs designed to form dsRNA in the plant cell, like hairpin, separate sense and antisense transgenes in one plant. This will result in better silencing of endogenous genes. This will result in continued transcription of silencing (dsRNA) molecules for trans-species RNA.
- Mexonized constructs may lead to higher efficiency in transgenic event selection. Less plants will have auto-silencing of the transgene resulting in higher protein expression levels, or better silencing of endogenous genes, or better trans-species silencing, or better promoter silencing (TGS) of endogenous genes (all depending on the purpose of the silencing construct).
- Durable expression levels of transgenes in genetic engineered animals. Protection of livestock against viruses, for instance the avian influenza H5N1, will depend on good and durable expression levels of RNAi effector molecules. Mexonized constructs will provide more stable expression through the omittance of epigenetic modification and TGS of the nuclear transgene, due by the produced RNAi effector molecules
- Long-lasting therapeutic effects of treatments with genetic engineered stem cells to cure diseases, for instance, cancer or infectious diseases like HIV, will depend on good and durable expression of the transgene or RNAi silencing molecules. Mexonized transgenes or shRNA constructs will provide more stable expression through the omittance of epigenetic modification and TGS due by the transcript-derived siRNAs.

The methods of the invention can find practical application in any area of technology where it is desirable to inhibit transcriptional silencing in a plant or other eukaryotic cells. The methods of the invention further find practical application where it is desirable to enhance expression of transgenes in a plant. Particularly useful practical applications include, but are not limited to, (1) expression of proteins or polypeptides in a plant; (2) protecting plants against pest infestation; (3) functional genomics in plants and generally any application wherein transcriptional silencing needs to be controlled or prevented.

The methods of the invention further find practical application where it is desirable to enhance expression of transgenes in animals. Particularly useful practical applications include, but are not limited to, (1) protection of livestock against viruses or prions; (2) stable expression of RNAi in pigs for cell-based therapies and organ transplantation; (3) functional genomics in embryonic stem cells and generally any application wherein transcriptional silencing needs to be controlled or prevented.

The methods of the invention further find practical application where it is desirable to enhance expression of transgenes in human cells. Particularly useful practical applications include, but are not limited to, (1) long-lasting expression of transgenes or shRNA for stem cell therapy, for instance for treatment of cancer or virus infections such as HIV; (2) functional genomics in human embryonic stem cells and generally any application wherein transcriptional silencing needs to be controlled or prevented.

In another aspect, the present invention relates to methods for producing products. In particular the invention relates to a method for producing a product of interest comprising culturing any of the host cells comprising the polynucleotide, the DNA construct or the vector herein described so as to express the product of interest and recovering the product from the host cell culture. In cases wherein the product is produced or secreted by the host cell used, the method may comprise an additional step of recovering the product from the culture medium. Also encompassed herein is any product of interest obtainable by (or obtained by) these methods.

"Products" as used herein include but are not limited to peptides, polypeptides, proteins, protein fragments, immunogenic fragments, antibodies and antibody fragments.

According to other embodiments, the invention relates to host cells that are plant cells. As such, the invention relates to a plant cell comprising a polynucleotide as described above operably linked to a transcriptional regulatory region regulating the transcription of the polynucleotide. Alternatively, said polynucleotide is inserted downstream and in frame of a transcriptional regulatory region regulating the transcription of the polynucleotide. The transcriptionally regulatory comprises a promoter as already described earlier. Preferably said promoter is an RNA polymerase promoter.

In a specific embodiment, the invention relates to a plant transformed with any of the polynucleotides, the DNA constructs or the vectors of the invention.

A "plant" as used herein is any of various photosynthetic, eukaryotic, multicellular organisms of the kingdom *Plantae* characteristically producing embryos, containing chloroplasts, and having cellulose cell walls. A part of a plant, *i.e*., a "plant tissue" may be treated according to the methods of the present invention to produce a transgenic plant. Many suitable plant tissues can be transformed according to the present invention and include, but are not limited to, somatic embryos, pollen, leaves, stems, calli, stolons, microtubers, and shoots. Thus, the present invention envisions the transformation of angiosperm and gymnosperm plants such as acacia, alfalfa, apple, apricot, artichoke, ash tree, asparagus, avocado, banana, barley, beans, beet, birch, beech, blackberry, blueberry, broccoli, brussels sprouts, cabbage, canola, cantaloupe, carrot, cassava, cauliflower, cedar, a cereal, celery, chestnut, cherry, chinese cabbage, citrus, clemintine, clover, coffee, corn, cotton, cowpea, cucumber, cypress, eggplant, elm, endive, eucalyptus, fennel, figes, fir, geranium, grape, grapefruit, groundnuts, ground cherry, gum hemlock, hickory, kale, kiwifruit, kohlrabi, larch, lettuce, leek, lemon, lime, locust, pine, maidenhair, maize, mango, maple, melon, millet, mushroom, mustard, nuts, oak, oats, okra, onion, orange, an ornamental plant or flower or tree, papaya, palm, parsley, parsnip, pea, peach, peanut, pear, peat, pepper, persimmon, pigeon pea, pine, pineapple, plantain, plum, pomegranate, potato, pumpkin, radicchio, radish, rapeseed, raspberry, rice, rye, sorghum, , sallow, soybean, spinach, spruce, squash, strawberry, sugarbeet, sugarcane, sunflower, sweet potato, sweet corn, tangerine, tea, tobacco, tomato, trees, triticale, turf grasses, turnips, a vine, walnut, watercress, watermelon, wheat, yams, yew, and zucchini.

According to the present invention "plant tissue" also encompasses plant cells. Plant cells include suspension cultures, callus, embryos, meristematic regions, callus tissue, leaves, roots, shoots, gametophytes, sporophytes, pollen, seeds and microspores. Plant tissues may be at various stages of maturity and may be grown in liquid or solid culture, or in soil or suitable media in pots, greenhouses or fields. A plant tissue also refers to any clone of such a plant, seed, progeny, propagule whether generated sexually or asexually, and descendents of any of these, such as cuttings or seed.

In that respect, the invention encompasses a seed, tuber, or reproductive or propagation material of any of the plants obtainable by or obtained by the methods described herein, wherein said seed, tuber or reproductive or propagation material comprises said polynucleotide, said DNA construct, or said vector of the invention.

In other embodiments, the invention relates to a a method for producing transgenic plants comprising: (a) transforming a plant cell with a polynucleotide (or DNA construct or vector) as herein described, (b) regenerating a transgenic plant from said plant cell, and (c) growing the transformed plant under conditions suitable for the transcription of the polynucleotide (or polynucleotide comprised in the DNA construct or vector).

Alternatively, the invention relates to a method for producing a commodity product, comprising: a) providing a polynucleotide according to previous aspects; b) introducing said sequence into a plant cell; c) growing said plant cell under conditions suitable for generating a plant; and d) producing a commodity product from said plant or part thereof.

Also encompassed is any commodity product obtainable by (or obtained by) this method.

The present invention also relates to a method for making a product of interest in a plant comprising: (a) transforming a plant cell with a polynucleotide (or vector) as described herein wherein said polynucleotide is operably linked to an RNA polymerase promoter or wherein said polynucleotide is inserted downstream and in frame of a transcriptional regulatory region regulating the transcription of the polynucleotide, (b) regenerating a transgenic plant from said plant cell, and (c) isolating plant cells or plant parts comprising the product of interest from said plant.

In another embodiment relates the invention to a method for (improving) transcription of a transgene in a plant comprising: (a) transforming a plant cell with a polynucleotide (or vector) wherein said polynucleotide is operably linked to an RNA polymerase promoter or wherein said polynucleotide is inserted downstream and in frame of a transcriptional regulatory region regulating the transcription of the polynucleotide, and (b) regenerating a transgenic plant from said plant cell

The above method may further comprise the step of isolating plant cells or plant parts comprising the transcribed transgene or comprising the product encoded by the transgene from said plant. Also encompassed is any of the products obtainable by (or obtained by) these methods.

According to another embodiment, the invention relates to a method for producing an RNA transcript in a plant comprising: (a) transforming a plant cell with a polynucleotide herein described, wherein said polynucleotide is operably linked to an RNA polymerase promoter or wherein said polynucleotide is inserted downstream and in frame of a transcriptional regulatory region regulating the transcription of the polynucleotide; or transforming a plant cell with a DNA construct herein described, or a vector herein described; and (b) regenerating a transgenic plant from said plant cell. The method may further comprise isolating plant cells or plant parts comprising the RNA transcript or the double stranded RNA.

In another embodiment, the invention also relates to any RNA transcript obtainable by the method for producing RNA described herein.

Also encompassed are the transgenic plants obtainable by (or obtained by) the methods described herein, as well as any harvestable plant part from the transgenic plant of the previous aspect. Also encompassed is any product produced from the plants or transgenic plants, wherein said product is selected from a group consisting of food, feed, fiber, paper, meal, protein, starch, flour, silage, coffee, tea, and oil.

The present invention finds further application in methods for pest control.

For instance, in one embodiment, the invention relates to a method of inhibiting infection of a first organism by a target organism comprising:
(a) transforming a cell of said first organism with a polynucleotide as described herein, wherein said polynucleotide is operably linked to an RNA polymerase promoter or wherein said polynucleotide is inserted downstream and in frame of a transcriptional regulatory region regulating the transcription of the polynucleotide; or transforming a plant cell with a DNA construct or a vector as described herein ; and
(b) regenerating an organism from said cell,
and wherein said polynucleotide after transcription and splicing down regulates expression of a target gene in said target organism.

As used herein, the expression "inhibiting infection" also can be worded as "preventing further infection". For instance, an uninfected transgenic plant is infected by the target organism (e.g. the pest organism or the pathogen). The target organism will feed from the plant (in case the first organism is a plant), thereby ingesting the RNA produced from the polynucleotide (or vector or DNA construct) in the plant. The so-produced RNA molecules (e.g. double stranded RNA) will interfere with the expression of the target gene, thereby causing lethality and/or mortality in the target organism and/or preventing the target organism from further feeding on the plant. As a result, further infection and/or infestation and/or damage of the plant will be reduced or stopped.

In preferred embodiments, the RNA transcript is double stranded RNA molecule wherein a first strand comprises a sequence substantially identical to a 19 to 49, 19 to 39, 19 to 29, 19 to 25, 19 to 21; or 10 to 1000, 10 to 500, 10 to 50, 19 to 999, 19 to 499 or 19 to more than 1000 contiguous nucleotides of a target gene; or comprising a sequence that has at least 70%, preferably at least 75%, 80%, 85%, 90%, 95% or 99% sequence identity with a sequence comprising a 19 to 49, 19 to 39, 19 to 29, 19 to 25, 19 to 21; or 10 to 1000, 10 to 500, 10 to 50, 19 to 999, 19 to 499 or 19 to more than 1000 contiguous nucleotides of a target gene; and wherein a second strand comprising a sequence substantially complementary to the first strand. Optionally, the double stranded RNA has a single stranded overhang at either or both ends. The above described method results in the inhibition of the target gene by said double stranded RNA. The target gene may be a gene in a second (ie target) organism such as an organism that infests plants or feeds on the plant containing the polynucleotides or DNA constructs of the invention.In some embodiments of the invention, where it is envisaged to produce dsRNA molecules for use in human applications or in animal/veterinary applications, it may be preferable that the length of formed dsRNA after splicing of the transcribed RNA is not too long, to avoid aspecific biological effects, known as the interferon response induced by long dsRNA molecules, in particular by stretches of dsRNA that are longer than 30 base pairs. For instance, in these embodiments, it is more preferred that the length of dsRNA is smaller, for instance between 19 and 29 contiguous basepairs.

In other embodiments of the invention, where trangene expression is in plants, for instance for the production of dsRNA for endogenous gene silencing or for 'trans-species' pest control, the length of the formed dsRNA after splicing of the transcribed RNA is variable and can go up to 1000 or more contiguous basepairs.

The method of inhibiting infection and/or infestation and/or damaging of a first organism by a target organism as described above may further comprise the step of evaluating whether the first organism is infected by the target organism.

The present invention also relates to methods for making a plant resistant to pest infestation comprising a first step of transforming a plant cell with a DNA construct, wherein the exons upon transcription and splicing result in a double stranded RNA consisting of: (i) a first strand comprising a sequence substantially identical to a 19 to 49, 19 to 39, 19 to 29, 19 to 25, 19 to 21; or 10 to 1000, 10 to 500, 10 to 50, 19 to 999, 19 to 499 or 19 to more than 1000 contiguous nucleotides of a target gene; or comprising a sequence that has at least 70%, preferably at least 75%, 80%, 85%, 90%, 95% or 99% sequence identity with a sequence comprising a 19 to 49, 19 to 39, 19 to 29, 19 to 25, 19 to 21; or 10 to 1000, 10 to 500, 10 to 50, 19 to 999, 19 to 499 or 19 to more than 1000 contiguous nucleotides of a target gene, and; (ii) a second strand comprising a sequence substantially complementary to the first, and wherein the double stranded RNA optionally has a single stranded overhang at either or both ends, and wherein the double stranded RNA inhibits expression of the said target gene, said method further comprising the step of regenerating a transgenic plant from said plant cell. The method may comprise the further step of analyzing the plant for resistance against said pest. The present invention further relates to any transgenic, pest resistant plant obtainable by the method described.

According to another embodiment, the inventions relates to compositions for use as a pesticide. Said compositions (or pesticides) may comprise any of the transgenic plants described herein, or any harvestable plant part thereof, or any product or commodity product derived thereof.

### Specific embodiments of the invention

Additional specific embodiments of the invention are described in this section in the following paragraphs which are numbered, to allow more easily refering back to.
1. An isolated polynucleotide comprising a sequence (to be transcribed), said sequence consisting of introns and exons wherein the size of each of the exons is smaller than 60 nucleotides.
2. An isolated polynucleotide as described before in paragraph 1, wherein the number of nucleotides in each of the exons independently ranges from 1 to 60 nucleotides, , preferably from 1 to 50, 1 to 40, 1 to 30, 1 to 26, from 1 to 21, from 1 to 17, from 1 to 15, from 1 to 10 nucleotides, more preferably from 2 to 60, 2 to 50, 2 to 40, 2 to 30, 2 to 26, from 2 to 21, from 2 to 17, from 2 to 15, from 2 to 10 nucleotides, most preferably from 4 to 60, 4 to 50, 4 to 40, 4 to 30, 4 to 26, from 4 to 21, from 4 to 17, from 4 to 15, or from 4 to 10 nucleotides, most preferably from 5 to 20 or from 10 to 15 nucleotides.
3. An isolated polynucleotide as described before in paragraph 1 wherein the number of nucleotides in each of the exons is independently chosen from the group comprising 60, 59, 58, 57, 56, 55, 54, 53, 52, 51, 50, 49, 48, 47, 46, 45, 44, 43, 42, 41, 40, 39, 38, 37, 36, 35, 34, 33, 32, 31, 30, 29, 28, 27, 26, 25, 24 or 23 nucleotides, preferably 22, 21, 20, 19, 18, 17, 16 or 15 nucleotides, more preferably 14, 13, 12, 11, 10, 9, 8, 7, 6, 5, 4, 3, 2 or 1 nucleotide.
4. A DNA construct comprising a polynucleotide as described before in any of paragraphs 1 to 3 operably linked to a transcriptional regulatory region regulating the transcription of the polynucleotide.
5. The DNA construct as described before in paragraph 4, wherein said transcriptional regulatory region comprises a promoter.
6. The DNA construct as described before in paragraph 4 or 5, wherein the exons upon transcription and splicing encode a product of interest.
7. The DNA construct as described before in any of paragraphs 4 to 6, wherein the exons upon transcription and splicing result in a double stranded RNA consisting of:
   (a) a first strand comprising a sequence substantially identical to 19 to 49, 19 to 39, 19 to 29, 19 to 25, 19 to 21; or 10 to 1000, 10 to 500, 10 to 50, 19 to 999, 19 to 499 or 19 to more than 1000 contiguous nucleotides of a target gene;
      or comprising a sequence that has at least 70%, preferably at least 75%, 80%, 85%, 90%, 95% or 99% sequence identity with a sequence comprising a 19 to 49, 19 to 39, 19 to 29, 19 to 25, 19 to 21; or 10 to 1000, 10 to 500, 10 to 50, 19 to 999, 19 to 499 or 19 to more than 1000 contiguous nucleotides of a target gene, and
   (b) a second strand comprising a sequence substantially complementary to the first,
      and wherein the double stranded RNA optionally has a single stranded overhang at either or both ends,
      and wherein the double stranded RNA inhibits expression of the target gene,
      for instance, wherein the double stranded RNA is part of an antisense polynucleotide, shRNA, miRNA, siRNA, stRNA, mRNA or ribozyme.
8. An RNA transcript encoded by the DNA construct as described before in any of paragraphs 4 to 7.
9. A vector comprising the polynucleotide as described before in any of paragraphs 1 to 3 or the DNA construct as described before in any of paragraphs 4 to 7.
10. The vector as described before in paragraph 9 which is an expression vector.
11. A cell transformed with the polynucleotide as described before in any of paragraphs 1 to 3 or the DNA construct as described before in any of paragraphs 4 to 7, or the vector as described before in paragraph 9 or 10, wherein the cell is chosen from a prokaryotic cell, a bacterial cell, a yeast cell, an eukaryotic cell, a plant cell, an animal cell or a human cell.
12. The cell as described before in paragraph 11 which is a prokaryotic cell, such as a bacterial cell.
13. The cell as described before in paragraph 11 which is an eukaryotic cell, such as an animal cell, a human cell, a plant cell, a yeast cell or a cell of a cell line.
14. An eukaryotic cell comprising the polynucleotide as described before in any of paragraphs 1 to 3 or the DNA construct as described before in any of paragraphs 4 to 7 integrated in its chromosome.
15. A method for producing a product of interest comprising culturing the host cell as described before in paragraphs 12 or 13 so as to express the product of interest and recovering the product from the host cell culture.
16. A product as described before in interest obtainable by the method described in paragraph 15.
17. A plant cell comprising a polynucleotide as described before in any of paragraphs 1 to 3 operably linked to a transcriptional regulatory region regulating the transcription of the polynucleotide or wherein said polynucleotide is inserted downstream and in frame of a transcriptional regulatory region regulating the transcription of the polynucleotide.
18. A plant transformed with the polynucleotide as described before in any of paragraphs 1 to 3, the DNA construct as described before in any of paragraphs 4 to 7, or the vector as described before in paragraph 9 or 10.
19. The plant as described before in paragraph 18 which is chosen from the group comprising acacia, alfalfa, apple, apricot, artichoke, ash tree, asparagus, avocado, banana, barley, beans, beet, birch, beech, blackberry, blueberry, broccoli, brussels sprouts, cabbage, canola, cantaloupe, carrot, cassava, cauliflower, cedar, a cereal, celery, chestnut, cherry, chinese cabbage, citrus, clemintine, clover, coffee, corn, cotton, cowpea, cucumber, cypress, eggplant, elm, endive, eucalyptus, fennel, figes, fir, geranium, grape, grapefruit, groundnuts, ground cherry, gum hemlock, hickory, kale, kiwifruit, kohlrabi, larch, lettuce, leek, lemon, lime, locust, pine, maidenhair, maize, mango, maple, melon, millet, mushroom, mustard, nuts, oak, oats, okra, onion, orange, an ornamental plant or flower or tree, papaya, palm, parsley, parsnip, pea, peach, peanut, pear, peat, pepper, persimmon, pigeon pea, pine, pineapple, plantain, plum, pomegranate, potato, pumpkin, radicchio, radish, rapeseed, raspberry, rice, rye, sorghum, , sallow, soybean, spinach, spruce, squash, strawberry, sugarbeet, sugarcane, sunflower, sweet potato, sweet corn, tangerine, tea, tobacco, tomato, trees, triticale, turf grasses, turnips, a vine, walnut, watercress, watermelon, wheat, yams, yew, and zucchinia, preferably said plant is a potato plant.
20. A seed, tuber, or reproductive or propagation material as described before in the plant as described before in paragraph 18 or 19, wherein said seed, tuber or reproductive or propagation material comprises a polynucleotide as described before in any of paragraphs 1 to 3, or a DNA construct as described before in any of paragraphs 4 to 7.
21. The seed, tuber, or reproductive or propagation material as described before in paragraph 20 which is derived from a plant as defined in paragraph 19, preferably a potato plant.
22. A method for producing transgenic plants comprising
   (a) transforming a plant cell with a polynucleotide as described before in any of paragraphs 1 to 3, or a DNA construct as described before in any of paragraphs 4 to 7, or a vector as described before in paragraph 9 or 10,.
   (b) regenerating a transgenic plant from said plant cell, and
   (c) growing the transformed plant under conditions suitable for the transcription of said polynucleotide.
23. A transgenic plant obtainable by the method as described before in paragraph 22.
24. The transgenic plant as described before in paragraph 23 which is a plant as defined in paragraph 19, preferably a potato plant.
25. A product produced from the plant as described before in paragraph 18, 19, 23 or 24, wherein said product is selected from a group consisting of food, feed, fiber, paper, meal, protein, starch, flour, silage, coffee, tea, and oil.
26. A harvestable plant part from the transgenic plant as described before in 18, 19, 23 or 24.
27. A method for producing a commodity product, comprising:
   a) providing a polynucleotide as described before in any of paragraphs 1 to 3, or a DNA construct as described before in any of paragraphs 4 to 7, or a vector as described before in paragraph 9 or 10,
   b) introducing said polynucleotide, DNA construct or vector into a plant cell;
   c) growing said plant cell under conditions suitable for generating a plant; and
   d) producing a commodity product from said plant or part thereof.
28. A commodity product obtainable by the method as described before in paragraph 27.
29. A method for making a product as described before in interest in a plant comprising:
   (a) transforming a plant cell with a polynucleotide as described before in any of paragraphs 1 to 3, wherein said polynucleotide is operably linked to an RNA polymerase promoter or wherein said polynucleotide is inserted downstream and in frame of a transcriptional regulatory region regulating the transcription of the polynucleotide; or transforming a plant cell with a DNA construct as described before in any of paragraphs 4 to 7, or a vector as described before in paragraph 9 or 10,
   (b) regenerating a transgenic plant from said plant cell, and
   (c) isolating plant cells or plant parts comprising the product of interest from said plant.
30. A method for improving transcription as described before in a transgene in a plant comprising:
   (a) transforming a plant cell with a polynucleotide as described before in any of paragraphs 1 to 3, wherein said polynucleotide is operably linked to an RNA polymerase promoter or wherein said polynucleotide is inserted downstream and in frame of a transcriptional regulatory region regulating the transcription of the polynucleotide; or transforming a plant cell with a DNA construct as described before in any of paragraphs 4 to 7, or a vector as described before in paragraph 9 or 10; and
   (b) regenerating a transgenic plant from said plant cell.
31. A method for transcription of a transgene in a plant as described before in paragraph 30, said method further comprising the step of isolating plant cells or plant parts comprising the transcribed transgene or comprising the product encoded by the transgene from said plant.
32. A product produced from the method as described before in paragraph 31.
33. A method for producing an RNA transcript in a plant comprising:
   (a) transforming a plant cell with a polynucleotide as described before in any of paragraphs 1 to 3, wherein said polynucleotide is operably linked to an RNA polymerase promoter or wherein said polynucleotide is inserted downstream and in frame of a transcriptional regulatory region regulating the transcription of the polynucleotide; or transforming a plant cell with a DNA construct as described before in any of paragraphs 4 to 7, or a vector as described before in paragraph 9 or 10; and
   (b) regenerating a transgenic plant from said plant cell.
34. The method as described before in paragraph 33, wherein said RNA transcript is a double stranded RNA molecule, shRNA, miRNA, siRNA, stRNA, mRNA or ribozyme.
35. A transgenic plant obtainable by the method as described before in paragraph 33 or 34
36. The method as described before in paragraph 33 or 34 further comprising isolating plant cells or plant parts comprising the RNA transcript or the double stranded RNA.
37. An RNA transcript obtainable by the method as described before in paragraph 33, 34 or 36.
38. A harvestable plant part from the transgenic plant as described before in paragraph 35.
39. A method of inhibiting infection of a first organism by a target organism comprising:
   (a) transforming a cell of said first organism with a polynucleotide as described before in any of paragraphs 1 to 3, wherein said polynucleotide is operably linked to an RNA polymerase promoter or wherein said polynucleotide is inserted downstream and in frame of a transcriptional regulatory region regulating the transcription of the polynucleotide; or transforming a plant cell with a DNA construct as described before in any of paragraphs 4 to 7, or a vector as described before in paragraph 9 or 10; and
   (b) regenerating an organism from said cell,
      and wherein said polynucleotide after transcription and splicing down regulates expression of a target gene in said target organism.
40. A method of inhibiting infection of a first organism by a target organism as described before in paragraph 39, further comprising the step of evaluating whether the first organism is infected by the target organism.
41. A method for making a plant resistant to pest infestation comprising:
   (a) transforming a plant cell with a DNA construct as described before in paragraph 7, and
   (b) regenerating a transgenic plant from said plant cell
42. The method for making a plant resistant to pest infestation as described before in paragraph 41, said method further comprising the step of analyzing the plant for resistance against said pest.
43. A transgenic plant obtainable by the method as described before in paragraph 42.
44. A pesticide comprising a transgenic plant as described before in paragraph 43, or comprising a harvestable plant part thereof.
45. A method for making transgenic tissues, organs or animals (human or non-human) for organ or tissue production comprising:
   (a) transforming an animal cell or a cell from a cell line with a DNA construct as described before in paragraph 7, and
   (b) regenerating a transgenic tissue, organ or animal from said cell,
      wherein optionally the polynucleotide corresponds to a gene or protein that needs to be controlled in the transgenic tissue, organ or animal.
46. A method for producing an RNA transcript in an animal cell (human or non-human) or a cell from a cell line (human or non-human) comprising:
   (a) transforming an animal cell with a polynucleotide as described before in any of paragraphs 1 to 3, wherein said polynucleotide is operably linked to an RNA polymerase promoter or wherein said polynucleotide is inserted downstream and in frame of a transcriptional regulatory region regulating the transcription of the polynucleotide; or transforming a animal cell with a DNA construct as described before in any of paragraphs 4 to 7, or a vector as described before in paragraph 9 or 10; and
   (b) regenerating a transgenic tissue, organ or animal (human or non-human) from said cell.
47. The method as described before in paragraph 46, wherein said RNA transcript is a double stranded RNA molecule, shRNA, miRNA, siRNA, stRNA, mRNA or ribozyme.
48. A method for preventing TGS (transcriptional gene silencing) of a transgene in a plant comprising:
   (a) providing a polynucleotide consisting of a coding sequence to be transcribed optionally under the control of a transcriptional regulatory region,
   (b) introducing into the coding sequence splice intron sequences so that stretches of double stranded RNA formed from expression of the transcript are of a size not capable of base-pairing with the nuclear transgene expression cassette sequence necessary to conduct the DNA methylation process,
   (c) transforming a plant cell with the polynucleotide obtained in step (b) wherein said polynucleotide is operably linked to said transcriptional regulatory region or wherein said polynucleotide is inserted downstream and in frame of an (endogenous) transcriptional regulatory region regulating the transcription of the polynucleotide, and
   (d) Regenerating a transgenic plant from said plant cell
49. The method as described before in paragraph 48 further comprising the step of evaluating whether correct transcription and optionally translation of the transgene has occurred.
50. An isolated polynucleotide comprising a sequence (to be transcribed), said sequence consisting of introns and exons wherein the size of each of the exons is smaller than 60 nucleotides, preferably the number of nucleotides in each of the exons is independently chosen from the group comprising 60, 59, 58, 57, 56, 55, 54, 53, 52, 51, 50, 49, 48, 47, 46, 45, 44, 43, 42, 41, 40, 39, 38, 37, 36, 35, 34, 33, 32, 31, 30, 29, 28, 27, 26, 25, 24 or 23 nucleotides, preferably 22, 21, 20, 19, 18, 17, 16 or 15 nucleotides, more preferably 14, 13, 12, 11, 10, 9, 8, 7, 6, 5, 4, 3, 2 or 1 nucleotide.
51. An isolated polynucleotide according to paragraph 50, wherein the number of nucleotides in each of the exons independently ranges from 1 to 60 nucleotides, preferably from 1 to 50, 1 to 40, 1 to 30, 1 to 26, from 1 to 21, from 1 to 17, from 1 to 15, from 1 to 10 nucleotides, more preferably from 2 to 60, 2 to 50, 2 to 40, 2 to 30, 2 to 26, from 2 to 21, from 2 to 17, from 2 to 15, from 2 to 10 nucleotides, most preferably from 4 to 60, 4 to 50, 4 to 40, 4 to 30, 4 to 26, from 4 to 21, from 4 to 17, from 4 to 15, or from 4 to 10 nucleotides, most preferably from 5 to 20 or from 10 to 15 nucleotides.
52. A DNA construct comprising a polynucleotide of aspect 50 or 51 operably linked to a transcriptional regulatory region regulating the transcription of the polynucleotide.
53. The DNA construct of aspect 52, wherein the exons upon transcription and splicing encode a product of interest.
54. The DNA construct of aspect 52, wherein the exons upon transcription and splicing result in a double stranded RNA consisting of:
   (a) a first strand comprising a sequence substantially identical to 19 to 49, 19 to 39, 19 to 29, 19 to 25, 19 to 21; or 10 to 1000, 10 to 500, 10 to 50, 19 to 999, 19 to 499 or 19 to more than 1000 contiguous nucleotides of a target gene;
      or comprising a sequence that has at least 70%, preferably at least 75%, 80%, 85%, 90%, 95% or 99% sequence identity with a sequence comprising a 19 to 49, 19 to 39, 19 to 29, 19 to 25, 19 to 21; or 10 to 1000, 10 to 500, 10 to 50, 19 to 999, 19 to 499 or 19 to more than 1000 contiguous nucleotides of a target gene, and
   (b) a second strand comprising a sequence substantially complementary to the first strand, and wherein the double stranded RNA optionally has a single stranded overhang at either or both ends,
      and wherein the double stranded RNA inhibits expression of the target gene.
55. A vector comprising the polynucleotide of aspect 50 or 51 or the DNA construct of any of aspects 52 to 54.
56. A cell transformed with the polynucleotide of aspect 50 or 51 or the DNA construct of any of aspects 52 to 54, or the vector of aspect 6, wherein the cell is chosen from a prokaryotic cell, a bacterial cell, a yeast cell, an eukaryotic cell, a plant cell, an animal cell or a human cell.
57. A method for producing a product of interest comprising culturing the cell of aspect 56 so as to express the product of interest and recovering the product from the host cell culture.
58. A plant transformed with the polynucleotide of aspect 50 or 51, the DNA construct of any of aspects 52 to 54, or the vector of aspect 55.
59. A method for producing transgenic plants comprising
   (a) transforming a plant cell with a polynucleotide of aspect 50 or 51, or a DNA construct of any of aspects 52 to 54, or a vector of aspect 55,
   (b) regenerating a transgenic plant from said plant cell, and
   (c) growing the transformed plant under conditions suitable for the transcription of said polynucleotide.
60. A method for making a product of interest in a plant comprising:
   (a) transforming a plant cell with a polynucleotide of aspect 50 or 51, wherein said polynucleotide is operably linked to an RNA polymerase promoter or wherein said polynucleotide is inserted downstream and in frame of a transcriptional regulatory region regulating the transcription of the polynucleotide; or transforming a plant cell with a DNA construct of any of aspects 52 to 54, or a vector of aspect 55,
   (b) regenerating a transgenic plant from said plant cell, and
   (c) isolating plant cells or plant parts comprising the product of interest from said plant.
61. A method for improving transcription of a transgene in a plant comprising:
   (a) transforming a plant cell with a polynucleotide of aspect 50 or 51, wherein said polynucleotide is operably linked to an RNA polymerase promoter or wherein said polynucleotide is inserted downstream and in frame of a transcriptional regulatory region regulating the transcription of the polynucleotide; or transforming a plant cell with a DNA construct of any of aspects 53 to 54, or a vector of aspect 55; and
   (b) regenerating a transgenic plant from said plant cell.
62. A method for producing an RNA transcript in a plant comprising:
   (a) transforming a plant cell with a polynucleotide of aspect 50 or 51, wherein said polynucleotide is operably linked to an RNA polymerase promoter or wherein said polynucleotide is inserted downstream and in frame of a transcriptional regulatory region regulating the transcription of the polynucleotide; or transforming a plant cell with a DNA construct of any of aspects 52 to 54, or a vector of aspect 55; and
   (b) regenerating a transgenic plant from said plant cell,
      preferably, said RNA transcript is chosen from a double stranded RNA, shRNA, miRNA, siRNA, stRNA or ribozyme.
63. A method of inhibiting infection of a first organism by a target organism comprising:
   (a) transforming a cell of said first organism with a polynucleotide of aspect 50 to 51, wherein said polynucleotide is operably linked to an RNA polymerase promoter or wherein said polynucleotide is inserted downstream and in frame of a transcriptional regulatory region regulating the transcription of the polynucleotide; or transforming a plant cell with a DNA construct of any of aspects 52 to 54, or a vector of aspect 55; and
   (b) regenerating an organism from said cell,
      and wherein said polynucleotide after transcription and splicing down regulates expression of a target gene in said target organism.
64. A method for making a plant resistant to pest infestation comprising:
   (a) transforming a plant cell with a DNA construct of aspect 55, and
   (b) regenerating a transgenic plant from said plant cell
65. A pesticide comprising a transgenic plant of aspect 58 or obtained by the method of aspect 59 or 64, or comprising a harvestable plant part of said transgenic plant.
66. A method for preventing TGS (transcriptional gene silencing) of a transgene in a plant comprising:
   (a) providing a polynucleotide consisting of a coding sequence to be transcribed optionally under the control of a transcriptional regulatory region,
   (b) introducing into the coding sequence splice intron sequences so that stretches of double stranded RNA formed from expression of the transcript are of a size not capable of base-pairing with the nuclear transgene expression cassette sequence necessary to conduct the DNA methylation process,
   (c) transforming a plant cell with the polynucleotide obtained in step (b) wherein said polynucleotide is operably linked to said transcriptional regulatory region or wherein said polynucleotide is inserted downstream and in frame of an (endogenous) transcriptional regulatory region regulating the transcription of the polynucleotide, and
   (d) Regenerating a transgenic plant from said plant cell.

### Description of Figures

**Figure 1A**: Schematical representation of a genome-integrated transgene expression construct that is subject to transcriptional gene silencing. The transgene construct is present in the genome between the left (LB) and right (RB) T-DNA borders. Transcription of the transgene is under control of its transgene promoter (TP). A neighbouring endogenous plant promoter (PP) causes aberrant transcripts of the transgene construct. Double stranded RNA is formed through base pairing of the transgene transcript and the aberrant transcript. The activity of RNA-dependent RNA polymerases may represent another source of dsRNA production. dsRNA is recognized by dicer-like RNAse type III enzymes (DCL) and cleaved into small interfering RNAs (siRNA) that guide the RNA-induced silencing complex (RISC) to homologous RNA molecules leading to Post transcriptional gene silencing (PTGS) Alternatively, siRNA molecules guide the RNA-induced transcriptional gene silencing (RITS) to homologous DNA sequences in the nucleus resulting in DNA methylation of the transgene construct sequences and resulting in transcriptional gene silencing (TGS). T: transcription termination site.
**Figure 1B**: Schematical representation of a genome-integrated 'mexonized' transgene expression construct, resistant to transcriptional gene silencing. The mexonized construct is present in the genome between the left (LB) and right (RB) T-DNA borders. The mexonized construct consists of 'mini-exons' or mexons of 10-15 nucleotides through the insertion of introns. The transgene is transcribed under control of the transgene promoter and gives rise to a spliced messenger RNA. Aberrant transcripts of the transgene construct that are generated by a neighbouring endogenous plant promoters (PP) are no longer capable of base pairing with the transgene transcripts to form stretches of double stranded (dsRNA) longer than 12-15 nucleotides. Any dsRNA from the spliced transgene transcript, e.g. through activity of RNA dependent RNA polymerases, when processed by dicer-like RNAse typelll enzymes (DCL) will only share 10-15 basepair of sequence homology with the original nuclear transgene DNA and no longer induce DNA methylation and transcriptional gene silencing (TGS). T: transcription termination site; PTGS: post-transcriptional gene silencing.
**Figure 1C**: Schematical representation of a genome-integrated hairpin expression construct that is subject to transcriptional gene silencing. The hairpin construct is present in the genome between the left (LB) and right (RB) T-DNA borders. Transcription of the hairpin is under control of its transgene promoter (TP). The transcript forms a double stranded (dsRNA) hairpin induced by intramolecular base pairing between the sense and antisense target sequence. The dsRNA hairpin is recognized by dicer-like RNAse type III enzymes (DCL) and cleaved into small interfering RNAs (siRNA) that guide the RNA-inducing silencing complex (RISC) to homologous RNA molecules leading to Post transcriptional gene silencing (PTGS). Alternatively, siRNA molecules guide the RNA-induced transcriptional gene silencing (RITS) to homologous DNA sequences in the nucleus resulting in DNA methylation of the hairpin construct sequence and resulting in transcriptional gene silencing (TGS). T: transcription termination site.
**Figure 1D**: Schematical representation of a genome-integrated 'mexonized' hairpin expression construct, resistant to transcriptional gene silencing. The mexonized construct is present in the genome between the left (LB) and right (RB) T-DNA borders. The mexonized hairpin consists of 'mini-exons' or mexons of 10-15 nucleotides through the insertion of introns. The mexonized hairpin is transcribed under control of the transgene promoter and upon splicing generates the double stranded RNA (dsRNA) hairpin sequence induced by intramolecular base pairing between the spliced sense and spliced antisense target sequence.

Upon processing by dicer-like RNAse typelll enzymes (DCL), the resulting siRNAs will only share 10-15 nucleotides of sequence identity with the original nuclear mexonized hairpin DNA and no longer induce DNA methylation and transcriptional gene silencing (TGS). T: transcription termination site; PTGS: post-transcriptional gene silencing.

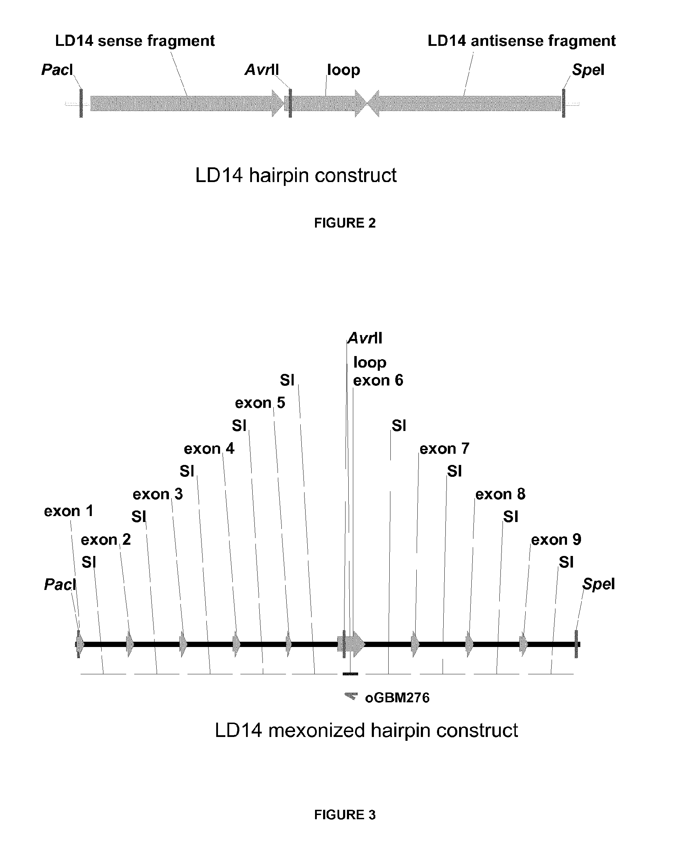

**Figure 2**: LD14 hairpin construct contains a sense 63 basepairs fragment of target LD14 from the Colorado potato beetle, a loop of 27 basepairs and the same 63 basepair fragment of target LD14 in antisense. This construct is present in the binary vector pGBM183.
**Figure 3**: LD14 mexonized hairpin construct contains a sense 63 basepairs fragment of target LD14, a loop of 27 basepairs and the same 63 basepair fragment of target LD14 in antisense. Exon sizes in sense and antisense fragments are kept small ranging between 9-16 basepairs through the insertion of synthetic introns (SI). This construct is present in the binary vector pGBM158.

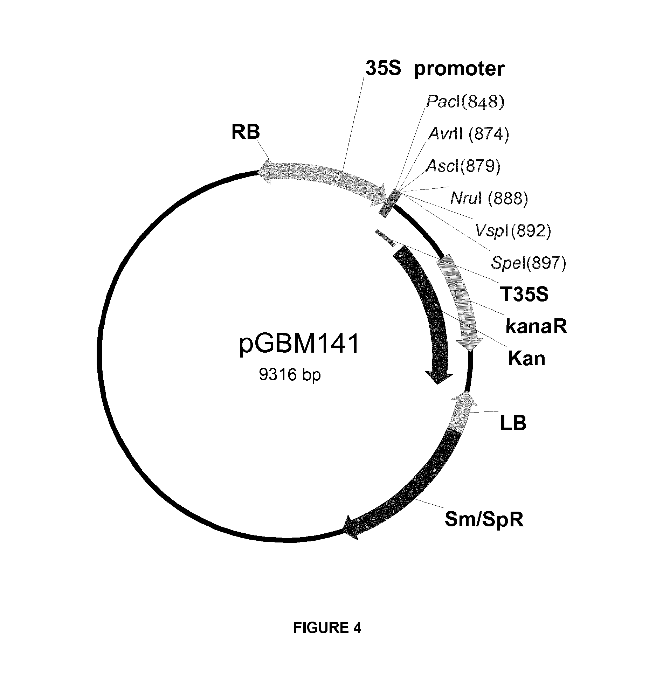

**Figure 4**: Binary vector pGBM141 for expression of transcripts under control of the 35S promoter

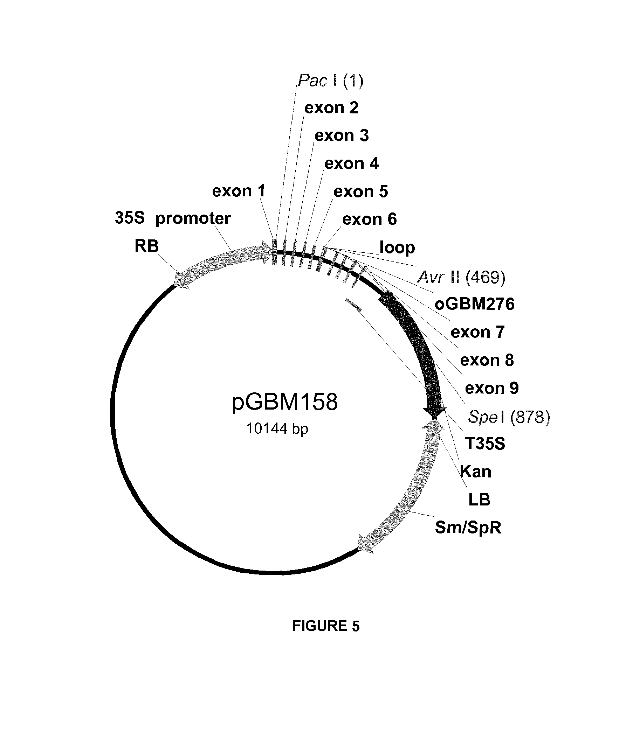

**Figure 5**: Binary vector pGBM158 for expression of the mexonized LD14 hairpin construct under control of the 35S promoter

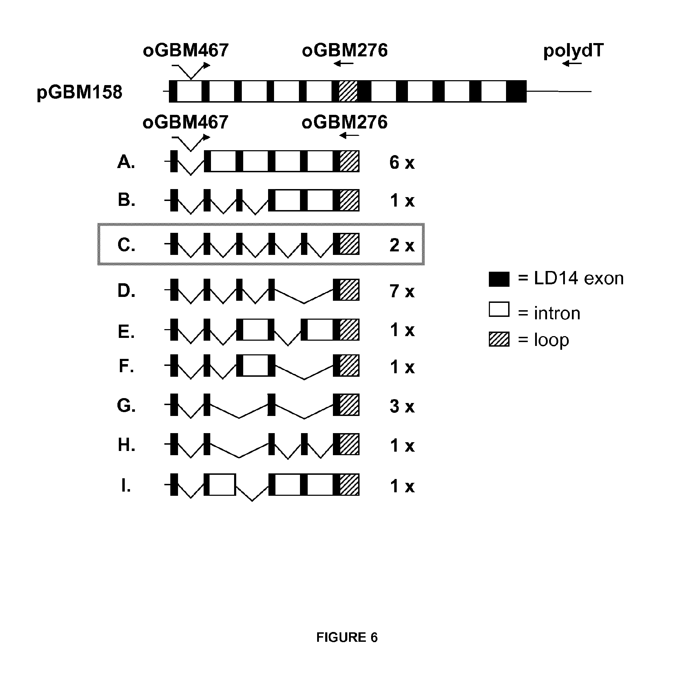

**Figure 6**: Schematical representation of transcript analysis of transgenic potato event P012/020. Transcripts were amplified from oligo-dT reverse transcribed RNA by PCR using forward primer oGBM467 and reverse primer oGBM276. The different transcripts obtained are represented in categories A till I and the corresponding frequency is indicated. In total, 23 transcripts were analyzed. PCR cloning vectors that contained transcript C and D were named pGBM267 and pGBM269 respectively.

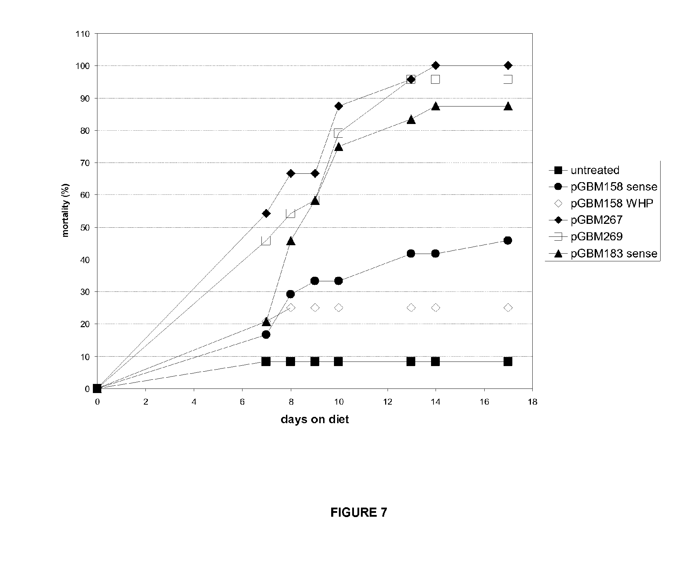

**Figure 7**: % mortality of Colorado potato beetles upon treatment with dsRNA topically applied to artificial diet in function of days on artificial diet. Untreated: water control. pGBM158 sense: dsRNA from sense fragment of pGBM158 mexonized insert. pGBM158 WHP: dsRNA from whole mexonized hairpin insert in pGBM158. pGBM267: dsRNA from cloned transcript of event P012/020 in pGBM267. pGBM269: dsRNA from cloned transcript of event P012/020 in pGBM269. pGBM183 sense: dsRNA from sense fragment of pGBM183 insert.

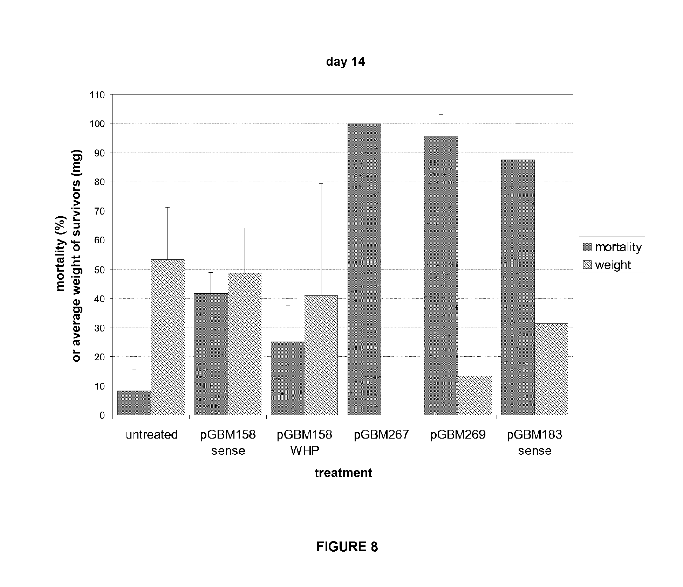

**Figure 8**: % mortality of Colorado potato beetles and average weight of surviving Colorado potato beetles at day 14 on artificial diet with topically applied dsRNA in function of treatment. Untreated: water control. pGBM158 sense: dsRNA from sense fragment of pGBM158 mexonized insert. pGBM158 WHP: dsRNA from whole mexonized hairpin insert in pGBM158. pGBM267: dsRNA from cloned transcript of event P012/020 in pGBM267. pGBM269: dsRNA from cloned transcript of event P012/020 in pGBM269. pGBM183 sense: dsRNA from sense fragment of pGBM183 insert.

The invention will be further understood with reference to the following non-limiting examples.

### Examples

### Example 1: Expression of a hairpin double stranded RNA of a target from Leptinotarsa decemlineata

### Construct design

A control RNAi construct was designed for the in planta expression of a hairpin RNA of a 63 bp target gene fragment (SEQ ID NO 3) derived from the *Leptinotarsa decemlineata* beetle. The target gene LD14 (partial cDNA sequence represented in SEQ ID NO 1) is an orthologue of the V-ATPase E subunit gene from Drosophila melanogaster (CG1088). In vitro prepared dsRNA of the selected fragment has shown to be insecticidal when fed to the *Leptinotarsa decemlineata* (the Colorado potato beetle) (see International application PCT/IB2006/003351 by applicant. The RNAi construct used in the present example contains a sense fragment and antisense fragment of SEQ ID NO 3. A loop of 27 nucleotides was included between the sense and antisense gene fragment to provide a restriction enzyme site Avrll and an annealing site for PCR primers. A Pacl restriction enzyme site was included at the 5' site of the sequence and a Spel restriction enzyme site was included at the 3' of the sequence for cloning purposes. The final sequence of this RNAi hairpin construct is given in SEQ ID NO 4 and graphically represented in Figure 2

A second construct similar to the previous construct was designed in which the exon sizes of the hairpin sequence have been kept small by insertion of introns every 10-16 nucleotides into the sense and antisense *Leptinotarsa decemlineata* sequences. As a source for introns, naturally occuring introns can be used, which can be retrieved from plant sequence databases or, alternatively, synthetic introns can be designed by someone skilled in the art, based on the consensus sequences of splice donor and acceptor sites, and the branch sites, as described in Lim and Burge (2001, PNAS. 98: 11193-11198). In this particular example, 9 synthetic introns have been designed and inserted in the sense and antisense fragment of the hairpin as indicated in Figure 3. The sequence of the 9 synthetic introns are given in SEQ ID NOS 5 to SEQ ID NO 13. Briefly, the designed synthetic introns were 80 bp long in size and were based on the consensus GT splice donor site, AG splice acceptor site and the most common branch sites as indicated in Lim and Burge (2001). To avoid repetitiveness, modifications were made in the branch site and also the position of the branch site was altered between the different synthetic introns. The remaining positions in the synthetic intron sequence were filled up with nucleotides, rich in % AT (∼70%) and pentameric sequences as described by Lim and Burge (2001). Additional AG and GT dinucleotides were avoided as such sequences may serve as cryptic splice donor or acceptor sites. The 'mexonized' hairpin construct with the small 'mini-exons' or 'mexons' and inserted introns is given in SEQ ID NO 14 and graphically represented in Figure 3. Similar as with the control construct, a loop of 27 nucleotides was included between the sense and antisense gene fragment to provide a restriction enzyme site Avrll and an annealing site for PCR primers. A Pacl restriction enzyme site was included at the 5' site of the sequence and a Spel restriction enzyme site was included at the 3' of the sequence for cloning purposes.

### In planta expression

The sequence of the LD14 hairpin construct and the LD14 mexonized construct were made synthetically and were cloned behind the 35S promoter into the binary vector pGBM141 (Figure 4) as a *PacI - SpeI* fragment. This binary vector was based on the pK7GWIWG2(I) (http://www.psb.ugent.be/gateway/index.php), received from the VIB (Vlaams Institute for Biotechnology, Gent) from which the gateway cloning system was replaced by a multiple cloning site, containing *PacI, Eco105I, AvrII, AscI, NruI, VspI* and *SpeI*.

The resulting vectors for the LD14 hairpin construct and the LD14 mexonized construct were named pGBM183 and pGBM158 respectively. The pGBM158 vector is graphically represented in Figure 5.

Stably transformed potato plants were obtained through Agrobacterium tumefaciens-mediated transformation of stem internode explants of potato 'Line V' (obtained from Wageningen University Department of Plant Sciences. obtained from the Laboratory of Plant Breeding at PRI Wageningen (Horsman, K. et al., 2001. Alteration of the genomic composition of Solanum nigrum potato backcross derivatives by somatic hybridization: selection of fusion hybrids by DNA measurements and GISH. Plant Breeding 120, pp. 201-207) which is derived from the susceptible diploid Solanum tuberosum 6487-9.

In vitro derived explants were inoculated with Agrobacterium tumefaciens C58C₁Rif^{R} containing the pGBM158 binary vector. After two days co-cultivation the explants were put onto a selective medium containing 100mg/l Kanamycin and 300mg/l Timentin. After 6 weeks post-transformation, the putative shoots were removed and rooted on selective medium.

The transgenic status of the rooting shoots was checked by PCR on extracted genomic DNA. PCRs were setup using the forward primer oGAU557 (SEQ ID NO 15) residing in the 35S promoter and using the reverse primer oGBM276 (SEQ ID NO 16) to score for presence or absence of the expression construct. Positive shoots were then clonally propagated in tissue culture and transplanted to soil. In total 27 events were obtained.

### Example 2: Molecular analysis of the transgenic events

The expression and splicing of the hairpin construct was analyzed by PCR. RNA was extracted from the transgenic potato event P012/020 and complementary DNA was synthesized using oligo dT as a primer for the reverse transcription reaction. PCR was conducted using the forward primer oGBM467 (SEQ ID NO 17) and reverse primer oGBM276 (SEQ ID NO 15) to amplify the part between the transcription start site and the hairpin loop (See FIGURE 6). The PCR fragments were separated by agarose gel electrophoresis, cut out at the size of 100 bp, 200,bp, 300 bp and 400 bp, purified from the gel and cloned into the TOPO-PCR4 cloning vector (Invitrogen). For each ligation, the sequence has been identified for the insert of 6 individual clones.

A total of 23 sequences was obtained. The sequences were analyzed by bioinformatics to identify the presence and/or absense of intron and exon sequences in the different transcripts. The sequences were analyzed with the EST2GENOME (EMBOSS) bioinformatics tool to identify intron and exon sequences. The obtained sequence was used as a query and the nuclear construct as present in pGBM158 was used as the genome sequence. An overview on presence or absence of intron and exon sequences is graphically represented in FIGURE 6. The results show that it is possible to obtain completely spliced transcripts from mexonized constructs. Through optimization of intron usage and/or insertion sites into the target sequence, or chosing different target gene fragments, the construct can be optimized towards 100% efficiency in splicing. A clone of transcript E and F were maintained as a source for dsRNA synthesis and subsequently analysis in the *Leptinotarsa decemlineata* bioassay. The clones were named pGBM267 and pGBM269 respectively.

### Example 3: Bioassay in Leptinotarsa decemlineata

The identified transcripts of the hairpin construct pGBN158 in the transgenic potato event P012/020 were used as a template to generate dsRNA in vitro. In addition, dsRNA was prepared from the following templates: pGBM158 sense fragment, pGBN 267 insert, pGBN269 insert, pGBN158 mexonized whole hairpin, pGBN183 whole hairpin control without introns. dsRNA was generated using the T7 Ribomax Express RNAi system kit (Promega) according to the manufacturer's protocol. The in vitro synthetized dsRNAs were tested for insecticidal activity in the Leptinotarsa decemlineata bioassay

Artificial diet for the Colorado potato beetle was prepared as follows (adapted from Gelman et al., 2001, J. Ins. Sc., vol. 1, no. 7, 1-10): water and agar were autoclaved, and the remaining ingredients (shown in Table 1 below) were added when the temperature was dropped to 50 °C. At this temperature, the ingredients were mixed well before the diet was aliquoted into 48-well plates (Nunc) with a quantity of 0.5 ml of diet per well. The artificial diet was allowed to soldify by cooling at room temperature. Diet was stored at 4 °C for up to three weeks.

**Table 1. Composition of artificial diet for bioassays of Leptinotarsa decemlineata larvae.**

| **ingredient** | **for 500 ml** |
|---|---|
| water | 384 ml |
| agar | 7 q |
| rolled oats | 20 g |
| Torula yeast | 30 g |
| lactalbumin hydrolysate | 15 g |
| casein | 5 g |
| fructose | 10 g |
| Wesson salt mixture | 29 g |
| tomato fruit powder | 6.25 g |
| potato leaf powder | 12.5 g |
| b-sitosterol | 0.5 g |
| sorbic acid | 0.4 g |
| methyl paraben (nipagin) | 1g |
| Vanderzant vitamin mix | 6 g |
| neomycin sulfate | 0.1 g |
| aureomycin | 0.065 g |
| rifampicin | 0.065 g |
| chlooramphenicol | 0.065 g |
| nystatin | 0.1 g |
| soybean oil | 1 ml |
| wheat germ oil | 1 ml |

Twenty-five µl of a solution of dsRNA at a concentration of 10 ng/µl was applied topically onto the solid artificial diet in the wells of the multiwell plate. The diet was dried in a laminair flow cabin. Per treatment, twenty-four Colorado potato beetle larvae (2^{nd} stage), with one insect per well (3 replicates with 8 larvae per replicate), were tested. The plates were stored in the insect rearing chamber at 25 ± 2 °C, ± 40 % relative humidity, with a 16:8 hours light:dark photoperiod.

The beetles were assessed as live or dead every 1, 2 or 3 days from day 7 onwards. The dsRNA from the different transcripts of the mexonized LD14 hairpin construct were compared to diet only (untreated) or diet with topically applied dsRNA corresponding to dsRNA from the sense fragment of the whole hairpin control construct in pGBM183 (SEQ ID NO 4), which served as the positive control and to the dsRNA from the sense and whole hairpin fragment of the LD14 mexonized whole hairpin construct in pGBM158 (SEQ ID NO 14).

Double-stranded RNA corresponding to the mexonized construct transcripts (i.e. present in pGBM267 and pGBM269) cloned from a transgenic potato event when fed to larvae of *Leptinotarsa decemlineata* resulted in significant increases in larval mortalities and reductions in weights of the survivors compared to dsRNA from the original nuclear sequence like pGBM158 sense and pGBM158 whole mexonized hairpin, as demonstrated in figures 7 and 8.

## Claims

1. A method for improving transcription of a transgene in a plant comprising:
(a) transforming a plant cell with:
(i) a polynucleotide comprising a sequence consisting of introns and exons wherein the size of each of the exons is smaller than 60 nucleotides;
(ii) a polynucleotide comprising a sequence consisting of introns and exons wherein the number of nucleotides in each of the exons independently ranges from 1 to 60 nucleotides,
wherein said polynucleotide is operably linked to an RNA polymerase promoter, or wherein said polynucleotide is inserted downstream and in frame of a transcriptional regulatory region regulating the transcription of the polynucleotide;
(iii) a DNA construct comprising the polynucleotide of (i) or (ii) operably linked to a transcriptional regulatory region regulating the transcription of said polynucleotide;
(iv) the DNA construct of (iii), wherein the exons upon transcription and splicing encode a product of interest;
(v) the DNA construct of (iii), wherein said exons upon transcription and splicing encode a double stranded RNA consisting of:
(1) a first strand comprising:
- a sequence substantially identical to 10 to 1000, 10 to 500, 10 to 50, 19 to 999, 19 to 499, 19 to 49, 19 to 39, 19 to 29, 19 to 25, 19 to 21 consecutive nucleotides of a target gene; or
- a sequence at least 70%, 75%, 80%, 85%, 90%, 95% or 99% sequence identity with a sequence comprising a 10 to 1000, 10 to 500, 10 to 50, 19 to 999, 19 to 499, 19 to 49, 19 to 39, 19 to 29, 19 to 25, 19 to 21 consecutive nucleotides of a target gene, and
(2) a second strand comprising a sequence substantially complementary to the first strand, wherein the double stranded RNA optionally has a single stranded overhang at either or both ends, and wherein the double stranded RNA inhibits expression of the target gene; or
(vi) a vector comprising the polynucleotide of (i) or (ii) or the DNA construct of any of (iii) to (v); and
(b) regenerating a transgenic plant from said plant cell.

2. A method for making a product of interest in a plant comprising:
(a) transforming a plant cell with:
(i) a polynucleotide comprising a sequence consisting of introns and exons wherein the size of each of the exons is smaller than 60 nucleotides;
(ii) a polynucleotide comprising a sequence consisting of introns and exons wherein the number of nucleotides in each of the exons independently ranges from 1 to 60 nucleotides,
wherein said polynucleotide is operably linked to an RNA polymerase promoter, or wherein said polynucleotide is inserted downstream and in frame of a transcriptional regulatory region regulating the transcription of the polynucleotide;
(iii) a DNA construct comprising the polynucleotide of (i) or (ii) operably linked to a transcriptional regulatory region regulating the transcription of said polynucleotide;
(iv) the DNA construct of (iii), wherein the exons upon transcription and splicing encode a product of interest;
(v) the DNA construct of (iii), wherein said exons upon transcription and splicing encode a double stranded RNA consisting of:
(1) a first strand comprising:
- a sequence substantially identical to 10 to 1000, 10 to 500, 10 to 50, 19 to 999, 19 to 499, 19 to 49, 19 to 39, 19 to 29, 19 to 25, 19 to 21 consecutive nucleotides of a target gene; or
- a sequence at least 70%, 75%, 80%, 85%, 90%, 95% or 99% sequence identity with a sequence comprising a 10 to 1000, 10 to 500, 10 to 50, 19 to 999, 19 to 499, 19 to 49, 19 to 39, 19 to 29, 19 to 25, 19 to 21 consecutive nucleotides of a target gene, and
(2) a second strand comprising a sequence substantially complementary to the first strand, wherein the double stranded RNA optionally has a single stranded overhang at either or both ends, and wherein the double stranded RNA inhibits expression of the target gene; or
(vi) a vector comprising the polynucleotide of (i) or (ii) or the DNA construct of any of (iii) to (v);
(b) regenerating a transgenic plant from said plant cell, and
(c) isolating plant cells or plant parts comprising the product of interest from said plant.

3. A method for preventing transcriptional gene silencing (TGS) of a transgene in a plant comprising:
(a) providing a polynucleotide consisting of a coding sequence to be transcribed optionally under the control of a transcriptional regulatory region,
(b) introducing into the coding sequence splice intron sequences to limit the exon sizes to less than 60 bp so that stretches of double stranded RNA formed from expression of the transcript are of a size not capable of base-pairing with the nuclear transgene expression cassette sequence necessary to conduct the DNA methylation process,
(c) transforming a plant cell with the polynucleotide obtained in step (b) wherein said polynucleotide is operably linked to said transcriptional regulatory region or wherein
said polynucleotide is inserted downstream and in frame of an (endogenous) transcriptional regulatory region regulating the transcription of the polynucleotide, and
(d) regenerating a transgenic plant from said plant cell.

4. A method for producing transgenic plants comprising
(a) transforming a plant cell with:
(i) a polynucleotide comprising a sequence consisting of introns and exons wherein the size of each of the exons is smaller than 60 nucleotides;
(ii) a polynucleotide comprising a sequence consisting of introns and exons wherein the number of nucleotides in each of the exons independently ranges from 1 to 60 nucleotides,
wherein said polynucleotide is operably linked to an RNA polymerase promoter, or wherein said polynucleotide is inserted downstream and in frame of a transcriptional regulatory region regulating the transcription of the polynucleotide;
(iii) a DNA construct comprising the polynucleotide of (i) or (ii) operably linked to a transcriptional regulatory region regulating the transcription of said polynucleotide;
(iv) the DNA construct of (iii), wherein the exons upon transcription and splicing encode a product of interest;
(v) the DNA construct of (iii), wherein said exons upon transcription and splicing encode a double stranded RNA consisting of:
(1) a first strand comprising:
- a sequence substantially identical to 10 to 1000, 10 to 500, 10 to 50, 19 to 999, 19 to 499, 19 to 49, 19 to 39, 19 to 29, 19 to 25, 19 to 21 consecutive nucleotides of a target gene; or
- a sequence at least 70%, 75%, 80%, 85%, 90%, 95% or 99% sequence identity with a sequence comprising a 10 to 1000, 10 to 500, 10 to 50, 19 to 999, 19 to 499, 19 to 49, 19 to 39, 19 to 29, 19 to 25, 19 to 21 consecutive nucleotides of a target gene, and
(2) a second strand comprising a sequence substantially complementary to the first strand, wherein the double stranded RNA optionally has a single stranded overhang at either or both ends, and wherein the double stranded RNA inhibits expression of the target gene; or
(vi) a vector comprising the polynucleotide of (i) or (ii) or the DNA construct of any of (iii) to (v);
(b) regenerating a transgenic plant from said plant cell, and
(c) growing the transformed plant under conditions suitable for the transcription of said polynucleotide.

5. A method for inhibiting expression of a target gene with double stranded RNA, wherein the double stranded RNA consists of:
(a) a first strand comprising:
- a sequence substantially identical to 10 to 1000, 10 to 500, 10 to 50, 19 to 999, 19 to 499, 19 to 49, 19 to 39, 19 to 29, 19 to 25, 19 to 21 consecutive nucleotides of a target gene; or
- comprising a sequence at least 70%, 75%, 80%, 85%, 90%, 95% or 99% sequence identity with a sequence comprising a 10 to 1000, 10 to 500, 10 to 50, 19 to 999, 19 to 499, 19 to 49, 19 to 39, 19 to 29, 19 to 25, 19 to 21 consecutive nucleotides of a target gene, and
(b) a second strand comprising a sequence substantially complementary to the first strand;
wherein the double stranded RNA optionally has a single stranded overhang at either or both ends; and
wherein said double stranded RNA is transcribed in an eukaryotic non-human cell from a DNA construct comprising:
- a polynucleotide comprising a sequence consisting of introns and exons, wherein the size of each of the exons is smaller than 60 nucleotides; or
- a polynucleotide comprising a sequence consisting of introns and exons wherein the number of nucleotides in each of the exons independently ranges from 1 to 60 nucleotides;
wherein the polynucleotide is operably linked to a transcriptional regulatory region regulating the transcription of said polynucleotide.

6. A method for producing a double stranded RNA comprising culturing an eukaryotic non-human cell transformed with:
(i) a DNA construct comprising:
- a polynucleotide comprising a sequence consisting of introns and exons wherein the size of each of the exons is smaller than 60 nucleotides, or
- a polynucleotide comprising a sequence consisting of introns and exons wherein the number of nucleotides in each of the exons independently ranges from 1 to 60 nucleotides;
wherein said polynucleotide is operably linked to a transcriptional regulatory region regulating the transcription of said polynucleotide, and
wherein said exons upon transcription and splicing encode a double stranded RNA consisting of:
(a) a first strand comprising:
- a sequence substantially identical to 10 to 1000, 10 to 500, 10 to 50, 19 to 999, 19 to 499, 19 to 49, 19 to 39, 19 to 29, 19 to 25, 19 to 21 consecutive nucleotides of a target gene; or
- a sequence at least 70%, 75%, 80%, 85%, 90%, 95% or 99% sequence identity with a sequence comprising a 10 to 1000, 10 to 500, 10 to 50, 19 to 999, 19 to 499, 19 to 49, 19 to 39, 19 to 29, 19 to 25, 19 to 21 consecutive nucleotides of a target gene, and
(b) a second strand comprising a sequence substantially complementary to the first strand;
and wherein the double stranded RNA optionally has a single stranded overhang at either or both ends and wherein the double stranded RNA inhibits expression of the target gene; or
(ii) a vector comprising the DNA construct of (i);
so as to express the double stranded RNA and recovering the product from the host cell culture.

7. A method for producing a product of interest comprising culturing an eukaryotic non-human cell transformed with:
(i) a polynucleotide comprising a sequence consisting of introns and exons wherein the size of each of the exons is smaller than 60 nucleotides;
(ii) a polynucleotide comprising a sequence consisting of introns and exons wherein the number of nucleotides in each of the exons independently ranges from 1 to 60 nucleotides,
wherein said polynucleotide is operably linked to an RNA polymerase promoter, or wherein said polynucleotide is inserted downstream and in frame of a transcriptional regulatory region regulating the transcription of the polynucleotide;
(iii) a DNA construct comprising the polynucleotide of (i) or (ii) operably linked to a transcriptional regulatory region regulating the transcription of said polynucleotide;
(iv) the DNA construct of (iii), wherein the exons upon transcription and splicing encode a product of interest; or
(v) a vector comprising the polynucleotide of (i) or (ii) or the DNA construct of (iii) or (iv);
so as to express the product of interest and recovering the product from the host cell culture.

8. The method according to any of claims 5 to 7, wherein said eukaryotic non-human cell is a plant cell.

9. A method for producing an RNA transcript in a plant comprising:
(a) transforming a plant cell with:
(i) a polynucleotide comprising a sequence consisting of introns and exons wherein the size of each of the exons is smaller than 60 nucleotides;
(ii) a polynucleotide comprising a sequence consisting of introns and exons wherein the number of nucleotides in each of the exons independently ranges from 1 to 60 nucleotides,
wherein said polynucleotide is operably linked to an RNA polymerase promoter or wherein said polynucleotide is inserted downstream and in frame of a transcriptional regulatory region regulating the transcription of the polynucleotide;
(iii) a DNA construct comprising the polynucleotide of (i) or (ii) operably linked to a transcriptional regulatory region regulating the transcription of said polynucleotide;
(iv) the DNA construct of (iii), wherein the exons upon transcription and splicing encode a product of interest;
(v) the DNA construct of (iii), wherein said exons upon transcription and splicing encode a double stranded RNA consisting of:
(1) a first strand comprising:
- a sequence substantially identical to 10 to 1000, 10 to 500, 10 to 50, 19 to 999, 19 to 499, 19 to 49, 19 to 39, 19 to 29, 19 to 25, 19 to 21 consecutive nucleotides of a target gene; or
- a sequence at least 70%, 75%, 80%, 85%, 90%, 95% or 99% sequence identity with a sequence comprising a 10 to 1000, 10 to 500, 10 to 50, 19 to 999, 19 to 499, 19 to 49, 19 to 39, 19 to 29, 19 to 25, 19 to 21 consecutive nucleotides of a target gene, and
(2) a second strand comprising a sequence substantially complementary to the first strand, wherein the double stranded RNA optionally has a single stranded overhang at either or both ends, and wherein the double stranded RNA inhibits expression of the target gene; or
(vi) a vector comprising the polynucleotide of (i) or (ii) or the DNA construct of any of (iii) to (v); and
(b) regenerating a transgenic plant from said plant cell,
wherein said RNA transcript is chosen from a double stranded RNA, shRNA, miRNA, siRNA, stRNA or ribozyme.

10. A method of inhibiting infection of a first non-human organism by a target organism comprising:
(a) transforming a cell of said first organism with:
(i) a polynucleotide comprising a sequence consisting of introns and exons wherein the size of each of the exons is smaller than 60 nucleotides;
(ii) a polynucleotide comprising a sequence consisting of introns and exons wherein the number of nucleotides in each of the exons independently ranges from 1 to 60 nucleotides, wherein said polynucleotide is operably linked to an RNA polymerase promoter or wherein said polynucleotide is inserted downstream and in frame of a transcriptional regulatory region regulating the transcription of the polynucleotide;
(iii) a DNA construct comprising the polynucleotide of (i) or (ii) operably linked to a transcriptional regulatory region regulating the transcription of said polynucleotide;
(iv) the DNA construct of (iii), wherein the exons upon transcription and splicing encode a product of interest;
(v) the DNA construct of (iii), wherein said exons upon transcription and splicing encode a double stranded RNA consisting of:
(1) a first strand comprising:
- a sequence substantially identical to 10 to 1000, 10 to 500, 10 to 50, 19 to 999, 19 to 499, 19 to 49, 19 to 39, 19 to 29, 19 to 25, 19 to 21 consecutive nucleotides of a target gene; or
- a sequence at least 70%, 75%, 80%, 85%, 90%, 95% or 99% sequence identity with a sequence comprising a 10 to 1000, 10 to 500, 10 to 50, 19 to 999, 19 to 499, 19 to 49, 19 to 39, 19 to 29, 19 to 25, 19 to 21 consecutive nucleotides of a target gene, and
(2) a second strand comprising a sequence substantially complementary to the first strand, wherein the double stranded RNA optionally has a single stranded overhang at either or both ends, and wherein the double stranded RNA inhibits expression of the target gene; or
(vi) a vector comprising the polynucleotide of (i) or (ii) or the DNA construct of any of (iii) to (v); and
(b) regenerating an organism from said cell,
wherein said polynucleotide after transcription and splicing down regulates expression of a target gene in said target organism.

11. A method for making a plant resistant to pest infestation comprising:
(a) transforming a plant cell with a DNA construct comprising:
(i) a polynucleotide comprising a sequence consisting of introns and exons wherein the size of each of the exons is smaller than 60 nucleotides;
(ii) a polynucleotide comprising a sequence consisting of introns and exons wherein the number of nucleotides in each of the exons independently ranges from 1 to 60 nucleotides,
wherein said polynucleotide is operably linked to an RNA polymerase promoter or wherein said polynucleotide is inserted downstream and in frame of a transcriptional regulatory region regulating the transcription of the polynucleotide;
(iii) a DNA construct comprising the polynucleotide of (i) or (ii) operably linked to a transcriptional regulatory region regulating the transcription of said polynucleotide;
(iv) the DNA construct of (iii), wherein the exons upon transcription and splicing encode a product of interest;
(v) the DNA construct of (iii), wherein said exons upon transcription and splicing encode a double stranded RNA consisting of:
(1) a first strand comprising:
- a sequence substantially identical to 10 to 1000, 10 to 500, 10 to 50, 19 to 999, 19 to 499, 19 to 49, 19 to 39, 19 to 29, 19 to 25, 19 to 21 consecutive nucleotides of a target gene; or
- a sequence at least 70%, 75%, 80%, 85%, 90%, 95% or 99% sequence identity with a sequence comprising a 10 to 1000, 10 to 500, 10 to 50, 19 to 999, 19 to 499, 19 to 49, 19 to 39, 19 to 29, 19 to 25, 19 to 21 consecutive nucleotides of a target gene, and
(2) a second strand comprising a sequence substantially complementary to the first strand, wherein the double stranded RNA optionally has a single stranded overhang at either or both ends, and wherein the double stranded RNA inhibits expression of the target gene; or
(vi) a vector comprising the polynucleotide of (i) or (ii) or the DNA construct of any of (iii) to (v); and
(a) regenerating a transgenic plant from said plant cell.

12. A pesticide comprising a transgenic plant obtained by the method of claim 4 or 11.

13. The method according to any one of the preceding claims, wherein the number of nucleotides in each of the exons is independently chosen from the group comprising 60, 59, 58, 57,56,55,54,53,52,51,50,49,48,47,46,45,44,43,42,41,40,39,38,37,36,35,34,33, 32, 31, 30, 29, 28, 27, 26, 25, 24, 23, 22, 21, 20, 19, 18, 17, 16, 15, 14, 13, 12, 11, 10, 9, 8, 7, 6, 5, 4, 3, 2 or 1 nucleotide(s), or wherein the number of nucleotides in each of the exons independently ranges from 1 to 60, 1 to 50, 1 to 40, 1 to 30, 1 to 26, 1 to 21, 1 to 17, 1 to 15, 1 to 10, 2 to 60, 2 to 50, 2 to 40, 2 to 30, 2 to 26, 2 to 21, 2 to 17, 2 to 15, 2 to 10, 4 to 60, 4 to 50, 4 to 40, 4 to 30, 4 to 26, 4 to 21, 4 to 17, 4 to 15, 4 to 10, 5 to 20, 9 to 16 or from 10 to 15 nucleotides.

14. The use of a dsRNA product produced by the method according to claim 6 or 13, for inhibiting expression of a target gene with said dsRNA.

15. The use of a dsRNA product produced by the method according to claim 6 or 13, for inhibiting infection of a first non-human organism by a target organism, wherein said dsRNA down regulates expression of a target gene in said target organism.

16. The use of a plant produced by the method according to claim 4 or 13, for producing a product of interest or a dsRNA.
